# EUROPEAN PATENT APPLICATION

(11) **EP 1 685 836 A2**
(43) Date of publication of application: **02.08.2006**
(21) Application number: 06075828.1
(22) Date of filing: 15.09.1999
(51) Int. Cl.: A61K 31/341, A61P 31/10, A61K 31/4184, A61K 31/5377, A61K 31/4439

(54) **Antifungal activity of dicationic molecules**

(30) Priority: 17.09.1998 US 100928 P
(62) Divisional of application: 99969025.8
(71) Applicant: UNIVERSITY OF NORTH CAROLINA AT CHAPEL HILL, Chapel Hill North Carolina 27599-4105 (US); The Georgia State University, Atlanta, GA 30303 (US); Duke University, Durham, NC 27712 (US)
(72) Inventor: Tidwell, Richard R., Pittsboro, NC 27312 (US); Boykin, David W., Atlanta, GA 30303 (US); Perfect, John R., Durham, NC 27707-3813 (US)
(74) Representative: Harrison Goddard Foote

(57) **Abstract**

Methods of treating fungal infections comprise administering a therapeutically effective amount of a compound described by Formulas [(I)-(II)]. Examples of fungal infections include *Candida albicans, Cryptococcus neoformans, Aspergillus fumigatus, Fusarium solani,* and combinations thereof.

## Description

The present invention was made with Government support under Grant Number 5-U19-A133363 from the National Institutes of Health. The Government has certain rights to this invention.

### Field of the Invention

The invention generally relates to methods for treating fungal infections.

### Background of the Invention

The incidence of fungal infections in the immunocompromised population has significantly increased over the past several years. In particular, *Candida* species, especially *Candida albicans*, are often significant pathogens in patients infected with human immunodeflcinecy virus (HIV). As an example, infections can range from somewhat mild oropharyngeal or vulvovaginal candidiasis to severe debilitating mucocutaneous candidiasis. Moreover, AIDS patients suffering from oral candidiasis may also experience esophageal candidiasis which has been known to lead to gastrointestinal bleeding and perforation. *Candida albicans* is a species which is commonly isolated from patients with the above-mentioned infections.

Treatment of candidiasis has typically involved two classes of drugs: (1) polyenes such as amphotericin B and nystatin; and (2) azoles such as clotrimazole, ketoconozole, fluconozole, and itraconazole. Since immunosuppression in AIDS-infected patients often occurs over an extended period of time, fungal reinfection may be common. Accordingly, these patients commonly receive prolonged antifungal therapy. Widespread antifungal therapy, however, has raised issues regarding the increased level of resistance among isolates of the *Candida* species, especially with respect to fluconozole. See Pfaller, M.A., et al., *Joumal of Clinical Microbiology,* Jan 1994, pp. 59-64; and Cameron, M.L., et al., *Antimicrobial Agents and Chemotherapy,* Nov. 1993, pp. 2449-2453.

In view of the above, there remains a need in the art to develop new antifungal treatment methods utilizing compounds which address the problems noted above. It would especially be desirable if the use of such compounds displayed increased fungal activity at acceptable dosage levels. Accordingly, it is an object of the present invention to provide new antifungal treatment methods exhibiting increased fungal activity at acceptable dosage levels.

### Summary of the Invention

The above object as well as others is provided by the present invention.
In one aspect, the invention provides a method of combating a fungal infection in a subject in need of such a treatment. The method comprises administering to the subject an effective fungal infection-combating amount of a compound [(I)-(VI)] selected from the group consisting of: 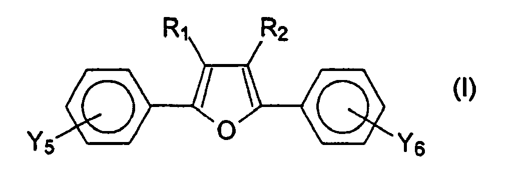 wherein R₁ and R₂ may be the same or different and selected from the group consisting of H, loweralkyl, aryl, alkylaryl, aminoalkyl, aminoaryl, halogen, oxyalkyl, oxyaryl, and oxyarylalkyl; and
wherein Y₅ and Y₆ are present in the meta or para positions and may the same or different and are represented by the formula (a) or (b) selected from the group consisting of: 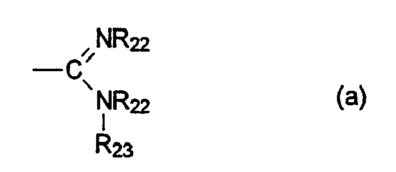 wherein:
each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
R₂₃ is H, hydroxy, loweralkyl, alkoxyalkyl, aminoalkyl, alkylamino, alkylaminoalkyl, cycloalkyl, hydroxycyctoalkyl, alkoxycycloalkyl, aryl, or alkylaryl; and 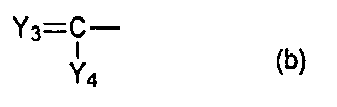 wherein Y₃ is selected from the group consisting of NR''' and O; wherein R''' is selected from the group consisting of H and loweralkyl;
   and wherein Y₄ is represented by the formula: 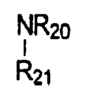 wherein R₂₀ is selected from the group consisting of H, loweralkyl, alkoxyalkyl, hydroxyalkyl, aminoalkyl, alkylaminoalkyl, cycloalkyl, aryl, or alkylaryl;
   wherein R₂₁ is selected from the group consisting of hydroxy, loweralkyl, alkoxyalkyl, hydroxyalkyl, aminoalkyl, alkylamino, alkylaminoalkyl, cycloalkyl, hydroxycycloalkyl, alkoxycycloalkyl, aryl, and alkylaryl; 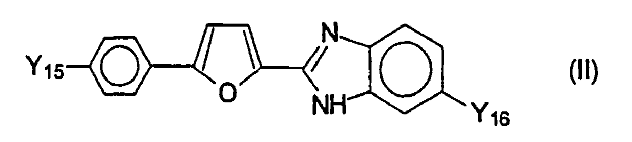 wherein Y₁₅ and Y₁₆ may be the same or different and represented by the formula: 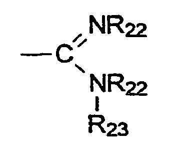 wherein:
   each R₂₂ is independently selected from the group consisting of H, loweralkyl, alkoxyalkyl, hydroxyalkyl, aminoalkyl, alkylaminoalkyl, alkylamino, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 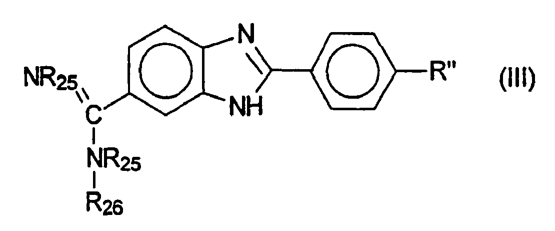 wherein each R₂₅ is independently selected from the group consisting of H, loweralkyl, alkoxyalkyl, hydroxyalkyl, aminoalkyl, alkylaminoalkyl, cycloalkyl, aryl, or alkylaryl or two R₂₅ groups together represent substituted or unsubstituted C₂-C₁₀ alkyl, hydroxyalkyl, or alkylene; and
   R₂₆ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   R" is hydroxy, alkoxyalkyl, hydroxyalkyl, alkoxyaryl, aryl, or the substituent selected from the formula (i) and (ii) consisting of: 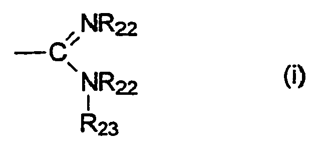 and 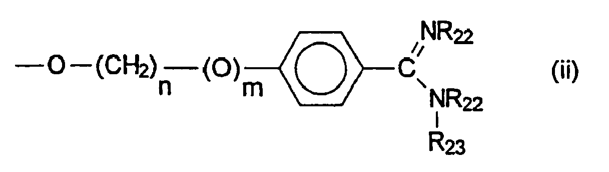
   wherein:
   n and m may be independently selected and each range from 0 to 6;
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 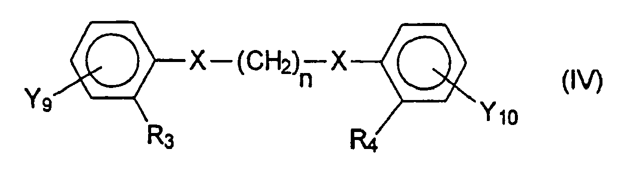
   wherein:
   n is from 2 to 6;
   X is selected from the group consisting of O, NH, and S;
   Y₉ and Y₁₀ may be in the meta or para position, are independently selected and are each represented by the formula: 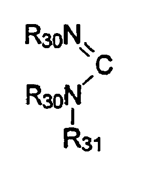
   wherein each R₃₀ is selected from the group consisting of H, hydroxy, loweralkyl, oxyalkyl, alkoxyalkyl, cycloalkyl, aryl, hydroxyalkyl, aminoalkyl, and alkylaminoalkyl; and wherein each of the two R₃₀ groups together may represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₃ and R₄ may be the same or different and are selected from the group consisting of H, amino nitro, loweralkyl, alkoxyalkyl, hydroxyalkyl, aminoalkyl, alkylaminoalkyl, cycloalkyl, aryl, or alkylaryl; 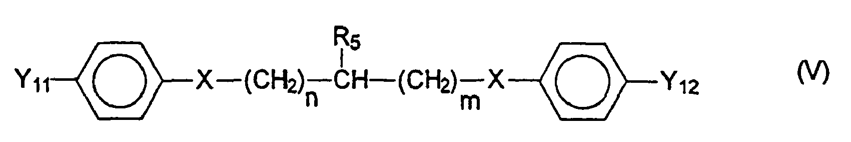 wherein X may be O, NH, or S; n and m may be the same or different and range from 2 to 6;
   wherein Y₁₁ and Y₁₂ may be the same or different and represented by the formula: 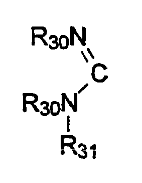 wherein each R₃₀ is selected from the group consisting of H, loweralkyl, oxyalkyl, alkoxyalkyl, cycloalkyl, aryl, hydroxyalkyl, aminoalkyl, and alkylaminoalkyl; and wherein each of the two R₃₀ groups together may represent represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₅ is selected from the group consisting of H, hydroxy, and 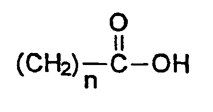 wherein n ranges from 0 to 3; 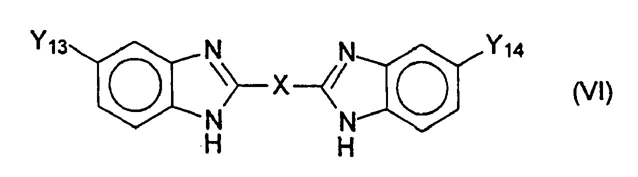 wherein X is C₁ to C₁₂ linear or branched, saturated or unsaturated alkyl containing up to four double bonds, or is substituted or unsubstituted aryl;
   wherein Y₁₃ and Y₁₄ may be the same or different and are represented by the formula: 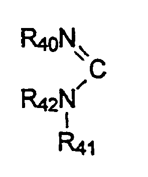 wherein R₄₀ and R₄₂ are each independently selected from the group consisting of H, loweralkyl, cycloalkyl, substituted aryl, and unsubstituted aryl, or wherein R₄₀ and R₄₂ together may represent represent C₂-C₁₀ alkyl, hydroxyalkyl, alkylene, substituted aryl, or unsubstituted aryl;
   and wherein R₄₁ may be H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.

In yet another aspect, the invention provides compounds for administering to a subject in need of fungal treatment. The compounds are represented by the formula: 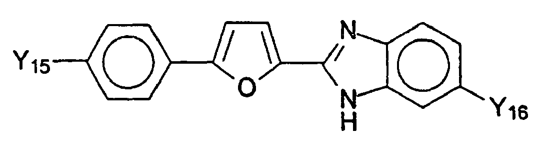 wherein Y₁₅ and Y₁₆ may be the same or different and represented by the formula: 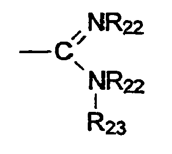 wherein:
each R₂₂ is independently selected from the group consisting of H, loweralkyl, alkoxyalkyl, hydroxyalkyl, aminoalkyl, alkylamino, alkylaminoalkyl, cycloalkyl, aryl, or alkylaryl or two R₂₂ groups together represent C₂-C₁₀ alkyl, hydroxyalkyl, or alkylene;
R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
and pharmaceutically acceptable salts thereof.

Further, the invention provides the above mentioned compounds and salts thereof as pharmaceutical formulations as described in greater detail herein.

The fungal infections that are addressed by the methods of the invention are generally selected from the group consisting of *Candida albicans, Cryptococcus neoformans, Aspergillus fumigatus, Fusarium solani,* and combinations thereof. Also, the methods of the invention may be used in combating *Candida* species other than *C*. *albicans* as well as fluconazole-resistant strains of *Candida albicans* and *Cryptococcus neoformans*.

The foregoing and other objects and aspects of the present invention are described in greater detail in the specification set forth hereinbelow.

### Detailed Description of the Preferred Embodiments

The present invention now will be described more fully hereinafter with reference to the accompanying specification and examples, in which preferred embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

As used herein, the term "lower alkyl" refers to C1 to C4 linear or branched alkyl, such as methyl, ethyl, propyl, butyl, isopropyl, sec-butyl, and tert-butyl. The term "cycloalkyl" as used herein refers to C3 to C6 cyclic alkyl, such as cyclopropyl, cyclobutyl, cyclopentyl, and cyclohexyl. The term "aryl" as used herein refers to C3 to C10 cyclic aromatic groups such as phenyl, naphthyl, and the like, and includes substituted aryl groups such as tolyl. The term "hydroxyalkyl" as used herein refers to C1 to C4 linear or branched hydroxy-substituted alkyl, i.e., -CH₂OH, -(CH₂)₂OH, etc. The term "aminoalkyl" as used herein refers to C1 to C4 linear or branched amino-substituted alkyl, wherein the term "amino" refers to the group NR'R", wherein R' and R" are independently selected from H or lower alkyl as defined above, i.e., -NH₂, - NHCH₃, -N(CH₃)₂, etc. The term "oxyalkyl" as used herein refers to C1 to C4 oxygen-substituted alkyl, i.e., -OCH₃, and the term "oxyaryl" as used herein refers to C3 to C10 oxygen-substituted cyclic aromatic groups. The term "lower alkoxy" as used herein refers to C1 to C4 linear or branched alkoxy, such as methoxy, ethoxy, propyloxy, butyloxy, isopropyioxy, and t-butyloxy. It should be appreciated that the various groups referred to above may be substituted or unsubstituted with various functional groups known to one skilled in the art.

As noted above, the methods of the present invention are useful for treating antifungal infections attributable to, for example, *Candida albicans, Cryptococcus neoformans, Aspergillus fumigatus, Fusarium solani,* and combinations thereof. The methods of the invention are useful for treating these conditions in that they inhibit the onset, growth, or spread of the condition, cause regression of the condition, cure the condition, or otherwise improve the general well-being of a subject inflicted with, or at risk of contracting the condition.

Subjects to be treated by the methods of the present invention are typically human subjects, although the methods of the present invention may be useful with any suitable subject known to those skilled in the art.

As noted above, the present invention provides pharmaceutical formulations comprising the aforementioned active compounds, or pharmaceutically acceptable salts thereof, in pharmaceutically acceptable carriers for oral, intravenous, or aerosol administration as discussed in greater detail below. Also, the present invention provides such compounds or salts thereof which have been lyophilized and which may be reconstituted to form pharmaceutically acceptable formulations for administration, as by intravenous or intramuscular injection.

The therapeutically effective dosage of any specific compound, the use of which is in the scope of present invention, will vary somewhat from compound to compound, patient to patient, and will depend upon the condition of the patient and the route of delivery. As a general proposition, a dosage from about 0.1 to about 50 mg/kg will have therapeutic efficacy, with all weights being calculated based upon the weight of the active based, including the cases where a salt is employed. Toxicity concerns at the higher level may restrict intravenous dosages to a lower level such as up to about 10 mg/kg, with all weights being calculated based upon the weight of the active base, including the cases where a salt is employed.. A dosage from about 10 mg/kg to about 50 mg/kg may be employed for oral administration. Typically, a dosage from about 0.5 mg/kg to 5 mglkg may be employed for intravenous or intramuscular injection. The duration of the treatment is usually once per day for a period of two to three weeks or until the fungal infection is essentially controlled. Lower doses given less frequently can be used to prevent or reduce the incidence of recurrence of the infection.

In accordance with the present method, pharmaceutically active compounds as described herein, or pharmaceutically acceptable salts thereof, may be administered orally as a solid or as a liquid, or may be administered intramuscularly or intravenously as a solution, suspension, or emulsion. Alternatively, the compounds or salts may also be administered by inhalation, intravenously or intramuscularly as a liposomal suspension. When administered through inhalation the active compound or salt should be in the form of a plurality of solid particles or droplets having a particle size from about 0.5 to about 5 microns, preferably from about 1 to about 2 microns.

The present invention also provides a pharmaceutical composition suitable for intravenous or intramuscular injection. The pharmaceutical composition comprises a compound of Formulas (I) through (VI) described herein, or a pharmaceutically acceptable salt thereof, in any pharmaceutically acceptable carrier. If a solution is desired, water is the carrier of choice with respect to water-soluble compounds or salts. With respect to the water-insoluble compounds or salts, an organic vehicle, such as glycerol, propylene glycol, polyethylene glycol, or mixtures thereof, may be suitable. In the latter instance, the organic vehicle may contain a substantial amount of water. The solution in either instance may then be sterilized in any suitable manner, typically by filtration through a 0.22 micron filter. Subsequent to sterilization, the solution may be filled into appropriate receptacles, such as depyrogenated glass vials. Of course, the filling should be done by an aseptic method. Sterilized closures may then be placed on the vials and, if desired, the vial contents may be lyophilized.

In addition to compounds of Formula (I) through (VI) or their salts, the pharmaceutical compositions may contain other additives, such as pH-adjusting additives. In particular, useful pH-adjusting agents include acids, such as hydrochloric acid, bases or buffers, such as sodium lactate, sodium acetate, sodium phosphate, sodium citrate, sodium borate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multidose use. Of course, as indicated, the pharmaceutical compositions of the present invention may be lyophilized using techniques well known in the art

In yet another aspect of the present invention, there is provided an injectable, stable, sterile composition comprising a compound of Formula (I) through (Vl), or a salt thereof, in a unit dosage form in a sealed container. The compound or salt is provided in the form of a lyophilizate which is capable of being reconstituted with a suitable pharmaceutically acceptable carrier to form a liquid composition suitable for injection thereof into a subject. The unit dosage form typically comprises from about 10 mg to about 10 grams of the compound or salt. When the compound or salt is substantially water-insoluble, a sufficient amount of emulsifying agent which is physiologically acceptable may be employed in sufficient quantity to emulsify the compound or salt in an aqueous carrier. One such useful emulsifying agent is phosphatidyl choline.

Other pharmaceutical compositions may be prepared from the water-insoluble compounds disclosed herein, or salts thereof, such as aqueous base emulsions. In such an instance, the composition will contain a sufficient amount of pharmaceutically acceptable emulsifying agent to emulsify the desired amount of the compound or salt thereof. Particularly useful emulsifying agents include phosphatidyl cholines, and lecithin.

Further, the present invention provides liposomal formulations of the compounds disclosed herein and salts thereof. The technology for forming liposomal suspensions is well known in the art. When the compound or salt thereof is an aqueous-soluble salt, using conventional liposome technology, the same may be incorporated into lipid vesicles. in such an instance., due to the water solubility of the compound or salt, the compound or salt will be substantially entrained within the hydrophilic center or core of the liposomes. The lipid layer employed may be of any conventional composition and may either contain cholesterol or may be cholesterol-free. When the compound or salt of interest is water-insoluble, again employing conventional liposome formation technology, the salt may be substantially entrained within the hydrophobic lipid bilayer which forms the structure of the liposome. In either instance, the liposomes which are produced may be reduced in size, as through the use of standard sonication and homogenization techniques.

Of course, the liposomal formulations containing the compounds disclosed herein or salts thereof, may be lyophilized to produce a lyophilizate which may be reconstituted with a pharmaceutically acceptable carrier, such as water, to regenerate a liposomal suspension.

Pharmaceutical formulations are also provided which are suitable for administration as an aerosol, by inhalation. These formulations comprise a solution or suspension of a desired compound described herein or a salt thereof, or a plurality of solid particles of the compound or salt. The desired formulation may be placed in a small chamber and nebulized. Nebulization may be accomplished by compressed air or by ultrasonic energy to form a plurality of liquid droplets or solid particles comprising the compounds or salts. The liquid droplets or solid particles should have a particle size in the range of about 0.5 to about 10 microns, more preferably from about 0.5 to about 5 microns. The solid particles can be obtained by processing the solid compound or a salt thereof, in any appropriate manner known in the art, such as by micronization. Most preferably, the size of the solid particles or droplets will be from about 1 to about 2 microns. In this respect, commercial nebulizers are available to achieve this purpose. The compounds may be administered via an aerosol suspension of respirable particles in a manner set forth in U.S. Patent No. 5,628,984, the disclosure of which is incorporated herein by reference in its entirety.

Preferably, when the pharmaceutical formulation suitable for administration as an aerosol is in the form of a liquid, the formulation will comprise a water-soluble compound or a salt thereof, in a carrier which comprises water. A surfactant may be present which lowers the surface tension of the formulation sufficiently to result in the formation of droplets within the desired size range when subjected to nebulization.

As indicated, the present invention provides both water-soluble and water-insoluble compounds and salts thereof. As used in the present specification, the term "water-soluble" is meant to define any composition which is soluble in water in an amount of about 50 mg/mL, or greater. Also, as used in the present specification, the term "water-insoluble" is meant to define any composition which has solubility in water of less than about 20 mg/mL. For certain applications, water soluble compounds or salts may be desirable whereas for other applications water-insoluble compounds or salts likewise may be desirable.

Examples of compounds for use in the methods of the invention include, but are not limited to:
4-(N-cyclopentylamidino)-1,2-phenylene diamine hydrochloride
2,5-Bis[2-(5-amidino)benzimidazoyl]furan hydrochloride
2,5-Bis[2-{5-(2-imidazolino)}benzimidazoyl]furan hydrochloride
2,5-Bis[2-(5-N-isopropylamidino)benzimidazoyl]furan hydrochloride
2,5-Bis[2-(5-N-cyclopentylamidino)benzimidazoyl]furan hydrochloride
2,5-Bis[2-(5-amidino)benzimidazoyl]pyrrole hydrochloride
2,5-Bis[2-{5-(2-imidazolino)}benzimidazoyl]pyrrole hydrochloride
2,5-Bis[2-(5-N-isopropylamidino)benzimidazoyl]pyrrole hydrochloride
2,5-Bis[2-(5-N-cyclopentylamidino)benzimidazoyl)pyrrole hydrochloride
1-Methyl-2,5-Bis[2-(5-amidino)benzimidazoyl]pyrrole hydrochloride
2,5-Bis[2-{5-(2-imidazolino)}benzimidazoyl]-1-methylpyrrole hydrochloride
2,5-Bis[2-(5-N-cyclopentyiamidino)benzimidazoyl] 1-methylpyrrole hydrochloride
2,5-Bis[2-(5-N-isopropylamidino)benzimidazoyl]thiophene hydrochloride
2,6-Bis[2-{5-(2-imidazolino)}benzimidazoyl]pyridine hydrochloride
2,6-Bis[2-(5-amidino)benzimidazoyl]pyridine hydrochloride
4,4'-Bis[2-(5-N-isopropytamidino)benzimidazoyl]-1,2-diphenylethane hydrochloride
4,4'-Bis[2-(5-N-cyclopentylamidino)benzimidazoyl]-2,5-diphenylfuran hydrochloride
2,5-Bis[2-(5-amidino)benzimidazoyl]benzo[b]furan hydrochloride
2,5-Bis[2-(5-N-cyclopentylamidino)benzimidazoyl]benzo[b]furan hydrochloride
2,7-Bis[2-(5-N-isopropylamidino)benzimidazoyl]fluorene hydrochloride
2,5-Bis-[4-(3-(N-morpholinopropyl)carbamoyl)phenyl]furan dihydrochloride
2,5-Bis[4-(2-N,N-dimethylaminoethylcarbamoyl)phenyl]furan dihydrochloride
2,5-Bis[4-(3-N,N-dimethylaminopropylcarbamoyl)phenyl]furan dihydrochloride
2,5-Bis[4-(3-N-methyl-3-N-phenylaminopropylcarbamoyl)phenyl]furan dihydrochloride
2,5-Bis[4-(3-N,N⁸,N¹¹-trimethylaminopropylcarbamoyl)phenyl]furan dihydrochloride
2,5-Bis[3-amidinophenyl]furan dihydrochloride
2,5-Bis-[3-(N-isopropylamidino)amidinophenyl]furan dihydrochloride
2,5-Bis[3[(N-(2-dimethylaminoethyl)amidino]phenylfuran dihydrochloride

As indicated, the compounds used in the present invention may be present as pharmaceutically acceptable salts. Such salts include the gluconate, lactate, acetate, tartarate, citrate, phosphate, borate, nitrate, sulfate, and hydrochloride salts.

The salts of the present invention may be prepared, in general, by reacting two equivalents of the pyrimidine base compound with the desired acid, in solution. After the reaction is complete, the salts are crystallized from solution by the addition of an appropriate amount of solvent in which the salt is insoluble.

Compounds employed in carrying out the methods of the present invention may be prepared in accordance with techniques known to those skilled in the art, particularly in light of the disclosure and examples set forth below. Exemplary techniques for preparing the compounds are set forth in U.S. Patent Nos. 5,602,172; 4,933,347; 5,723,495; 5,428,051; 5,643,935; 5,639,755; 4,963,589; and 5,578,631, the disclosures of which are incorporated by reference herein in their entirety. For example, the compounds described in Formula (I) can be prepared by: (a) cyclodehydrative furanization of 1,4-diketones according to the procedure taught by R.E. Lutz, et al., J. *Am. Chem. Soc.* **56:**2698 (1934) to form 2,5-bis-(4-bromophenyl) furan; (b) nitrilization of 2,5-bis(4-bromophenyl) furan using copper (I) cyanide to produce the corresponding bis-nitrile 2,5-bis-(4-cyanophenyl) furan; and (c) conversion of the bis-nitrile to the desired bis-dicationic aryl furan used in the invention.

According to a second method, compounds of Formula (I) may be prepared by (a) converting the appropriate bromoacetophenone to the ethyl bromophenyl-oxopropionate using sodium hydride and diethylcarbonate in tetrahydrofuran, (b) converting the ethyl bromophenyl-oxopropionate to the ethyl bis-bromobenzoyl- propionate using bromophenacyl bromide, (c) converting the bis-bromobenzoyl- propionate to the ethyl bis-bromophenyl furan using ethanol and hydrochloric acid, (d) hydrolysis of the ethyl bis-bromophenyl furan to the bis-bromophenyl furan carboxylic acid using potassium hydroxide followed by hydrochloric acid, (e) converting the carboxylic acid to the corresponding bis-nitrile with copper (I) cyanide and heat, and converting the bis-nitrile to the appropriate bis-dicationic aryl furan.

Conversion of the bis-nitrile to the bis-dicationic aryl furan of Formula (I) may be accomplished according to several methods known to those skilled in the art. According to one method, conversion of the bis-nitrile to the bis-dicationic aryl furan is carried out by conversion into intermediate imidate esters using classical Pinner methodology, followed by reaction of these intermediates with ammonia or the appropriate diamine for example, ethylenediamine, 1,3-propanediamine, etc. According to another method, the bis-nitrile is converted to the bis-dicationic aryl furan by fusion of the bis-nitrile directly with the hydrochloride salt of the appropriate diamine by thermolysis. This technique is particularly useful for the preparation of compounds wherein two R groups together form a cyclic alkyl.

Compounds described by Formulas (IV) and (V) can be prepared, for example, according to the following procedure with reference to Charts I-III described in U.S. Patent No. 4,933,347. Such compounds can also be synthesized according to techniques described in U.S. Patent No. 5,723,495. More specifically, the compounds may be prepared by first synthesizing known bis-nitriles using Allen's procedure for alkylation of phenols. See J.N. Ashley et al., *J. Chem. Soc*. 103-116 (1942); C.F.H. Allen et al., *Org. Synth. Coll*. III, (1955). The compounds can then be obtained by using variations of the known techniques of Clement and Raether and by using appropriate reagents. See B. Clement and W. Raether, *Arzneim. Forsch.* 35, 1009-1014 (1985).

### Examples

The invention will now be described in greater detail with reference to the following examples. It should be noted that these examples are for illustrative purposes only, and are not meant to limit the invention.

Compounds used in the methods of the invention were synthesized in the laboratories of the inventors. The synthesis and physical properties for the following compounds have been previously described as follows : Compounds 1-4 & 7-14, 16-20 (Tidwell, R.R., J. *Med. Chem.,* 33: pp. 1252-1257 (1990)); Compound 15 (Jones, S.K., et al., *Antimicrob. Agents Chemother.* 34: pp. 1026-1030 (1990)); Compounds 21-27 (Berger, B.J., et al., J. *Pharmacol. Exp. Therapeut* 256: pp. 883-889 (1991)); Compounds 28, 30, 32, and 47 (Fairley, T., et al., J. *Med. Chem.,* 36: pp. 1746-1753 (1993); Tidwell, R.R., et al., J. *Med. Chem.* 21: pp. 613-623 (1978)); Compounds 29, 31, 33, 34, and 48-50 (Fairley, T., et al., J. *Med. Chem.,* **36:** pp. 1746-1753 (1993)). The synthesis of the remaining compounds, 35-46, and 51-57, along with their physical properties are described below.

The compound melting points were recorded using a Thomas Hoover (Uni-Melt) capillary melting point apparatus and were not corrected. Proton (¹H) and Carbon (¹³C) Nuclear Magnetic Resonance (NMR) spectra of the compounds were recorded employing a Varian GX400 spectrometer and chemical shifts (d) are given in ppm relative to tetramethylsilane (TMS). Coupling constants (J) are reported in Hertz. Mass spectra (MS) were recorded on a VG Instruments 70-SE spectometer (Georgia Institute of Technology, Atlanta, GA). IR spectra were recorded using a Michelson 100 (Bomen, Inc.) instrument. Elemental analysis was obtained from Atlantic Microlab In. (Norcross, GA) and are with in ± 0.4 of the theoretical values. All chemicals and solvents were purchased from Aldrich Chemical Co., St. Louis, MO or Fisher Scientific, Pittsburgh, PA.

### Example 1

### 4-(N-cyclopentytamidino)-1,2-phenylene diamine hydrochloride

This compound is used as an intermediate in the synthesis of compounds 38, 42, 45, 54, and 56. Distilled cyclopentylamine (1.83 g, 0.021 mol) was added to a stirred suspension of the imidate ester hydrochloride (4.91 g, 0.02 mol) formed from 4-cyano-2-nitroaniline under Pinner type conditions in 30 ml of dry ethanol, the mixture was stirred for 12 h at room temperature and for 1 h at 50°C. The solvent was removed under reduced pressure and the residual thick oily mass was triturated with dry ether and dried under vacuum to yield 4.7 g (95%), mp 168-179°C dec. ¹H NMR (DMSO-d₆): 9.29 (br, 3H), 8.45(d, 2H, J = 2.4), 803 (s, 2H), 7.77(dd.1H, J = 2.4, 8.8), 4.20 (quintet, 1 H, J = 6.0), 2.04-2.0 (m, 2H), 1.73-1.65 (m, 4H), 1.57-1.49 (m. 2H). ¹³C NMR (DMSO-d₆): 160.4, 148.5, 134.1, 129.4, 127.3, 118.9, 114.6, 54.1, 31.2, 23.5. The 4-(N-cyclopentylamidino)-2-nitroaniline (5.0g, 0.02 mol) mp 238-240°C dec; used directly without further characterization (5.0 g, 0.02 mol) and 1.0 g of 10% Pd/C in 130 ml of dry methanol was subjected to hydrogenation at 50 psi for approximately 1 h. The catalyst was filtered over Celite, washed with hot methanol and the solvent of the filtrate was removed under reduced pressure, the residue was triturated with dry ether, and the solid was filtered and dried under vacuum at 45°C for 24 h. The yield of light brown hygroscopic solid was 3.91 g (72%); The compound yielded the following physical properties: mp 170-178°C. ¹H NMR (DMSO-d₆): 8.97 (br s, 1H), 8.82 (br s, 1H), 8.64 (br s, 1 H), 6.89 (s, 1H), 6.88 (d, 1H, J = 8.4), 6.59 (d, 1 H, J = 8.4), 5.40 (br, 2H), 5.0 (br, 2H) 4.17 (m, 2H), 2.10-1.98 (m, 2H), 1.82-1.76 (m, 4H). ¹³C NMR (DMSO-d₆): 162.4, 140.8, 134.0, 118.4, 115.6, 113.0, 112.5, 53.7, 31.3, 23.5. MS(FAB) 219 (M⁺+1). Anal. calc. for C₁₂H₁₈N₄·HCl·H₂O): C, 52.84; H, 7.76; N, 20.54. Found: C, 53.10;H, 7.77; N, 20.72.

### Example 2

### 2,5-Bis[2-(5-amidino)benzimidazoyl] furan hydrochloride (Compound 35).

A solution of furan-2,5-dicaboxaldehyde (S. Morikawa, (1979) "Synthesis of hydroxymethylfural and 2,5-furandicarboxaldehydes", *Noguchi kenkyusho Jiho,* 21: pp. 25-33) (0.8 g 2 mmol), 4-amidino-1,2-phenylene diamino hydrochloride hydrate (0.8 g, 4 mmol) and 1,4-benzoquinone (0.432 g, 4 mmol) in ethanol (40 ml) was heated at reflux for 4 h (under nitrogen) (Bajic, M. et al., *Hetero. Com.* **1:** 225-230 (1995)). The reaction mixture was cooled to room temperature and the dark solid was collected by filtration, washed with cold ethanol and then anhydrous ether, and dried to yield 0.55 g (71%) of the free base. This solid was dissolved slowly in hot ethanol (300 ml) and filtered. The filtrate volume was reduced to 70 ml and acidified with HCl saturated ethanol. After standing overnight in the refrigerator, the green solid was collected by filtration, washed with anhydrous ether and dried under vacuum to yield 0.4 g (52%) yield of solid. The compound demonstrated the following physical properties: mp > 300°C.¹H NMR(DMSO-d₆): 9.30 (s, 4H); 8.19 (2s, 2H), 7.81, (d, 2H, J = 8.8), 7.72 (d, 2H, J = 8.4). 7.60 (s, 2H). ¹³C NMR (DMSO-d₆/D₂O): 166.8, 146.4, 146.1, 142.2, 139.7, 123.4, 122.7, 117.1, 116.1, 115.4, MS (FAB): m/z 385 (M⁺+1); HRMS: calc. mass (free base) 385.1525 (M⁺+1); observed mass: 385.1535. Anal. calc. for C₂₀H₁₆N₈O•2HCl•1.5 H₂O: C, 49.59; H, 4.37; N, 23.14. Found: C, 49.40; H, 4.31; N, 22.96.

### Example 3

### 2,5-Bis[2-{5-(2-imidazolino)}benzimidazoyl]furan hydrochloride (Compound 36).

A procedure similar to that described above was employed for the condensation of 2,5-furandicarboxaldehyde (S. Morikawa, (1979) "Synthesis of hydroxymethylfural and 2,5-furandicarboxaldehydes", *Noguchi kenkyusho Jiho,* 21: pp. 25-33) and 2-(3,4-diaminophenyl)imidazoline (Fairley, T., (1993) *J. Med. Chem.,* 36: 1746-1753; Tidwell, R.R., (1978) J. *Meal Chem.,* 21:613-623) to give a 38% yield of a green powder. This compound demonstrated the following physical properties: mp <300°C, ¹H NMR (DMSO-d₆): 10.53 (s,4H), 8.38 (s, 2H), 7.87 (d, 2H, J = 8.5), 7.83(d, 2H, J = 8.2), 7.62 (s, 2H), 4.04(s, 8H). ¹³MR (DMSO-d₆/D₂O): 166.3, 146.2, 146.1, 142.3, 139.8, 123.7, 117.6, 116.9, 116.1, 115.5, 45.0. MS (FAB): m/z 437 (M⁺+1); HRMS: calc. mass (free base): 437.1838 (M⁺+1); observed mass: 437.1832. Anal. calc. for C₂₄H₂₀N₈O•2HCl•5H₂O: C, 48.08; H, 5.38; N, 18.69. Found: C, 48.22; H, 5.25; N, 18.51.

### Example 4

### 2,5-Bis[2-(5-N-isopropylamidino)benzimidazoyl]furan hydrochloride (Compound 37).

A procedure similar to that described above was employed for the condensation of 2,5-furandicarboxaldehyde (S. Morikawa, (1979) "Synthesis of hydroxymethylfural and 2,5-furandicarboxaldehydes", *Noguchi kenkyusho Jiho,* 21: pp. 25-33) and 4-(N-isopropylamidino)-1,2-phenylene diamine (Fairley, T., (1993) *J. Med. Chem.,* **36**: 1746-1753; Tidwell, R.R., (1978) *J. Med. Chem.,* 21:613-623) to give a 54% yield of a yellow-green powder. This compound displayed the following physical properties: mp > 300°C.¹H NMR (DMSO-d₆): 9.60 (s, 1 H), 9.58 (s, 1H), 9.45 (s, 2H), 9.04 (s, 2H), 8.06 (s, 2H), 7.82 (d, 2H, J = 8.4), 7.69(s, 2H), 7.62 (d, 2H, J = 8.2), 4.09 (m, 2H, J = 7.0), 1.32 (d, 12H, J = 6.3). ¹³C NMR (DMSO-d₆/D₂O): 162.8, 145.9, 145.1, 140.9, 138.5, 124.5, 124.0, 116.9, 115.9, 115.8, 45.9, 21.7. MS (FAB): m/z 469 (M⁺+1); HRMS: calc. mass (free base): 469.2464 (M⁺+1); observed mass: 469.2475. Anal. calc. for: C₂₆H₂₈N₈O•3HCl•2.5H₂O: C, 50.12; H, 5.83; N, 17.99. Found: C, 50.45; H, 5.76; N, 17.64.

### Example 5

### 2,5-Bis[2-(5-N-cyclopentylamidino)benzimidazoyl]furan hydrochloride (Compound 38).

A procedure similar to that described above in the previous examples was employed for the condensation of 2,5-furandicarboxaldehyde and 4-(N-cyclopentylamidino)-1,2-phenylene diamine to give a 77% yield of a yellow-green powder. The following physical properties were obtained: mp 287-289 °C dec. ¹H NMR (DMSO-d₆'D₂O): 8.07 (s, 2H), 7.82 (d, 2H, J = 8.4), 7.66 (s, 2H), 7.63 (d, 2H, J = 8.4), 4.22-4.14 (m, 2H), 2.14- 2.04 (m, 4H), 1.82-1.67 (m, 8H), 1.64-1.56 (m, 4H).¹³C NMR (DMSO-d₆): 163.0, 145.4, 144.5, 140.4, 137.7, 123.9, 116.4, 115.5, 115.2, 54.6, 31.5, 23.7. MS (FAB) 521 (M⁺+1). Anal. calc. for C₃₀H₃₂N₈O•4HCl : C, 54.06; H, 5.44; N, 16.81. Found: C, 53.80; H, 5.51; N, 16.68.

### Example 6

### 2,5-Bis[2-(5-amidino)benzimidazoyl]pyrrole hydrochloride (Compound 39).

A procedure similar to that described in the above examples was employed for the condensation of 4-amidino-1,2-phenylene diamino hydrochloride hydrate (Fairley, T., (1993) *J. Med Chem.,* 36: 1746-1753; Tidwell, R.R., (1978) J. *Med* Chem., 21:613-623) with pyrrole-2,-5-dicarboxaldehyde (Miller, R., (1981) *Acta Chem. Scand.,* B: 303-304) to yield 0.83 g (76%) of solid. The compound displayed the following physical properties: mp > 300°C. ¹H NMR (DMSO-d₆): 9.48 (br s, 1H), 9.18 (br s, 1H), 8.25 (s, 2H), 7.87 (d, J = 8.4, 2H), 7.80 (dd, J = 8.8 and 0.8, 2H). 7.54 (s, 2H). MS (free base): m/e 384 (M⁺+1). Anal., calc. for C₂₀H₁₇N₉•3HCl•3H₂O: C, 43.93; H, 4.73; N, 23.05. Found: C, 43.61; H, 4.62; N, 22.79.

### Example 7

### 2,5-Bis[2-{5-(2-imidazolino)}benzimidazoyl]pyrrole hydrochloride (Compound 40).

A procedure similar to that described in the above examples was used for the condensation of pyrrole-2,5-dicarboxaldehyde and 2-(3,4-diaminophenyl)imidazoline (Fairley, T., (1993) *J. Med. Chem.,* **36:** 1746-1753; Tidwell, R.R., (1978) *J. Med. Chem.,* 21:613-623) to give a 86% yield of solid. The compound dispalyed the following physical properties: mp > 300 °C. ¹H NMR (DMSO-d₆): 10.71 (s, 1 H), 8.44 (s, 2H), 7.92 (dd, J = 8.4 and 1.6 , 2H), 7.86 (d, J = 8.8 , 2H), 7.39 (s, 2H), 4.04 (s, 8H). MS(free base): m/e 436 (M⁺+1). Anal. calc. for C₂₄H₂₁N₉•3HCl•4H₂O: C, 46.72; H, 5.23; N, 20.43. Found: C, 46.49; H, 5.11; N, 20.28.

### Example 8

### 2,5-Bis[2-(5-N-isopropylamidino)benzimidazoyl]pyrrole hydrochloride (Compound 41).

A procedure similar to that described in the above examples was used for the condensation of pyrrole-2,5-dicarboxaldehyde and 4-(N-isopropylamidino)-1,2-phenylene diamine (Fairley, T., (1993) *J. Med. Chem.,* **36**: 1746-1753; Tidwell, R.R., (1978) *J. Med. Chem.,* **21**:613-623) to yield (79%) of a yellow-green solid. The compound displayed the following physical properties: mp 287-289°C dec. ¹H NMR (DMSO-d₆/D₂O): 8.06 (s, 2H), 7.81 (d, 2H, J = 8.4), 7.65 (d, 2H, J = 8.4), 7.41 (s, 2H), 4.06 (septet, 2H, J = 6.4), 1.30 (d, 12H, J = 6.4). ¹³C NMR (DMSO-d₆/D₂O): 162.3, 145.8, 138.6, 135.7, 124.7, 124.2, 123.9, 115.7, 115.5, 114.9, 45.7, 21.4. MS (FAB) 468 (M⁺+1). Anal. calc. for C₂₆H₂₉N₉•4HCl: C, 50.90; H, 5.42; N, 20.55. Found: C 51.54; H 5.57; N 20.30.

### Example 9

### 2,5-Bis[2-(5-N-cyclopentylamidino)benzimidazoyl]pyrrole hydrochloride (Compound 42).

A procedure similar to that described in the above examples was employed for the condensation of 4-(N-cyciopentylamidino)-1,2-phenylene diamine with pyrrole-2,-5-dicarboxaldehyde (Miller, R., (1981) *Acta Chem. Scand.,* B: 303-304) to yield (71%) a blue-green solid. The compound displayed the following physical properties: mp 290- 294°C. ¹H NMR (DMSO-d₆/D₂O): 8.0 (s, 2H), 7.77(d, 2H, J = 8.4), 7.60 (d, 2H, J = 8.4), 7.32 (s, 2H), 4.09 (br m, 2H), 2.11-1.97 (m, 4H), 1.77-1.62 (m, 8H), 1.61-1.50 (m, 4H). ¹³C NMR (DMSO-d₆/D₂O): 163.1, 145.7, 138.6, 135.6, 124.6, 124.3, 123.8, 115.8, 115.5, 115.1, 55.0, 31.7, 23.9. MS (FAB) m/z 520 (M⁺+1). Anal. calc. for C₃₀H₃₃N₉•4HCl•0.5 H₂O: C, 53.42; H, 5.63; N, 18.68. Found: C 53.90; H 5.75; N 18.16.

### Example 10

### 1-Methyl-2,5-Bis[2-(5-amidino)benzimidazoyl]pyrrole hydrochloride (Compound 43).

A procedure similar to that described in the above examples was employed for the condensation of 4-amidino-1,2-phenylene diamine hydrochloride hydrate (Fairley, T., (1993) J. *Med. Chem.,* 36: 1746-1753; Tidwell, R.R., (1978) J. *Med. Chem.,* 21:613-623) with 1-methylpyrrole-2,-5-dicarboxaldehyde (Cresp, T.M., (1973) J. *Chem. Soc., Perkin Trans.* 1: 2961-2971) to give a 70 % yield of product. The compound displayed the following physical properties: mp>300°C; ¹H NMR (DMSO-d₆): 9.38 (br s, 1H), 9.11 (br s, 1H), 8.19 (s, 2H), 7.80 (d, J = 8.4), 7.73 (dd, J = 8 and 1.2, 2H), 7.33 (s, 2H), 4.72 (s, 3H). MS(free base): m/z 398 (M⁺+1). Anal. calc. for C₂₁H₁₉N₉•3HCl•H₂O: C, 48.06; H, 4.61; N, 24.02. Found: C, 48.16; H, 4.58; N, 23.93.

### Example 11

### 2,5-Bis[2-{5-(2-imidazolino)}benzimidazoyl]-1-methylpyrrole hydrochloride (Compound 44).

A procedure similar to that described in the above examples was employed for the condensation of 2-(3,4-diaminophenyl)imidazoline (Fairley, T., (1993) *J. Med. Chem.,* 36: 1746-1753; Tidwell, R.R., (1978) J. *Med Chem.,* 21:613-623) with 1-methylpyrrole-2,-5-dicarboxaldehyde. A yield of 83% of solid mp > 300°C was obtained. The following additional physical properties were determined: ¹H NMR(DMSO-d₆): 10.60 (s, 1H), 8.36 (s, 2H), 7.84 (dd, J = 8.4 and 8, 4H), 7.30 (s, 2H), 4.72 (s, 3H), 4.04 (s, 8H). MS(free base): mle 450 (M⁺+1). Anal. calc. for C₂₅H₂₃N₉•3HCl•3H₂O: C, 48.98; H, 5.26; N, 20.57. Found: C, 49.20; H, 4.79; N, 20.51.

### Example 12

### 2,5-Bis[2-(5-N-cyclopentylamidino)benzimidazoyl] 1-methylpyrrole hydrochloride (Compound 45).

A procedure similar to that described in the above examples was employed for the condensation of 4-(N-cyclopentylamidino)-1,2-phenylene diamine with 1-methyl-2,-5-pyrrole dicarboxaldehyde (Cresp, T.M., (1973) *J*. *Chem. Soc., Perkin Trans.* 1: 2961-2971) to yield (85%) a blue solid having a mp of 324-326°C. The following additional physical properties were determined: dec. ¹H NMR (DMSO-d₆/D₂O): 8.0 (s, 2H), 7.73 (d, 2H, J = 8.4), 7.55 (d, 2H, J = 8.4), 7.13 (s, 2H), 4.57 (s, 3H), 4.14 (quintet, 2H, J = 5.2), 2.12-2.02 (m, 4H), 1.80-1.58 (m, 12H). MS (FAB) 534 (M⁺+1). Anal. calc. for C₃₁H₃₅N₉•3HCl•H₂O, C: 56.32; H, 6.10; N, 19.07. Found: C 56.90; H 5.97; N 18.83.

### Example 13

### 2,5-Bis[2-(5-N-isopropylamidino)benzimidazoyl]thiophene hydrochloride (Compound 46).

A procedure similar to that described in the above examples was employed for the condensation of 2,5-thiophenedicarboxaldehyde and 4-(N-isopropylamidino)-1,2-phenylene diamine (Fairley, T., (1993) *J. Med. Chem.,* 36:1746-1753; Tidwell, R.R., (1978) *J*. *Med. Chem.,* 21:613-623) to give a 75% yield of a green yellow solid mp 290-292 °C. The physical properties for this compound were determined to be: dec. ¹H NMR (DMSO-d₆/D₂O): 8.18 (s, 2H), 8.05 (s, 2H), 7.77 (d, 2H, J = 8.4); 7.60 (d, 2H, J = 8.4), 4.11 (quintet, 2H, J = 6.4), 1.31 (d, 12H, J = 6.4). MS (FAB) m/z 485 (M⁺+1). Anal. calc. for C₂₆H₂₈N₈S•3HCl•H₂• . C, 51.02; H, 5.43; N, 18.31; CI, 17.38. Found: C 51.56; H 5.54; N 18.09; Cl, 17.37.

### Example 14

### 2,6-Bis[2-{5-(2-imidazolino)}benzimidazoyl]pyridine hydrochloride (Compound 51).

A procedure similar to that described in the above examples was used for the condensation of 2,6-pyridine carboxyaldehyde and 2-(3,4-diaminophenyl)imidazoline (Fairley, T., (1993) J. *Med. Chem.,* 36: 1746-1753; Tidwell, R.R., (1978) *J. Med Chem.,* 21:613-623) to give an 85% yield of solid mp > 300°C. The physical properties for this compound were found to be: ¹H NMR (DMSO-d,): 10.71 (s, 1 H), 8.51-8.49 (m, 4H), 8.30 (m, 1 H), 7.96(m, 4H), 4.05(s, 8H). MS (free base): m/e 448(M⁺+1). Anal. calc. for C₂₅H₂₁N₉•3HCl•3H₂O: C, 49.15; H, 4.94; N, 20.63. Found: C, 49.14; H, 4.68; N, 20.51.

### Example 15

### 2,6-Bis[2-(5-amidino)benzimidazoyl]pyridine hydrochloride (Compound 52).

A procedure similar to that described in the above examples was used to condense 2,6-pyridine dicarboxaldehyde with 4-amidino-1,2-phenylene diamine hydrochloride hydrate (Fairley, T., (1993) *J. Med. Chem.,* 36: 1746-1753; Tidwell, R.R., (1978) J. *Med. Chem.,* 21:613-623) to yield 89% of a solid mp > 300 °C. The physical properties for this compound were determined to be: ¹H NMR (DMSO-d₆): 9.45 (br s, 1H), 9.12 (br s, 1H), 8.51 (d, J = 8 , 2H), 8.34-8.28 (m, 3H), 7.94 (d, J = 8.4 , 2H), 7.79 (dd, J = 8.4 and 1.6, 2H). MS(free base): m/z 396 (M⁺+1). Anal. calc. for C₂₁H₁₇N₉•3HCl•3H₂O: C, 45.13; H, 4.69; N, 22.56. Found: C, 45.16; H, 4.58; N, 22.45.

### Example 16

### 4,4'-Bis[2-(5-N-isopropylamidino)benzimidazoyl]-1,2-diphenytethane hydrochloride (Compound 53).

1,2-Bis-(4-cyanophenyl)ethane was prepared in one step from 2,3-bis-(4-bromophenyl)propanoic acid (Dann, O., (1971) *Liebigs Ann. Chem.,* 749: 68-89) by the action of CuCN in DMF (Das, B.P., et al., (1977) *J. Med. Chem., 20:* 531-536) in a 50% yield; mp 195-197 °C. ¹H NMR (DMSO-d₆): 7.68 (d, 4H, J = 8), 7.40 (d, 4H, J = 8), 3.01(s, 4H). ¹³NMR (DMSO-d₆): 146.7, 131.9, 129.3, 118.6, 108.6, 35.8. MS m/e 232 (M⁺+1). 1,2-Bis-(4-cyanophenyl)ethane was used without further characterization and on treatment with DIBAL gave a white crystalline solid (CHCl₃ : ether) 76% mp 121-122 °C of 1,2-bis-(4-formylphenyl)ethane. ¹H NMR (DMSO-d₆): 9.96 (s, 2H), 7.80 (d, 4H, J = 8), 7.44 (d, 4H J = 8), 3.05 (s, 4H). ¹³C NMR (DMSO-d₆ / D₂O): 192.0, 184.0, 134.2, 129.1, 128.8, 36.0. Anal. Calc. for C₁₆H₁₄O₂₀•0.1 H₂O: C, 80.04; H, 5.96. Found: C, 80.09; H, 5.98. A protocol similar to that described above was employed for the condensation of 1,2-bis-(4-formylphenyl)ethane and 4-(N-isopropylamidino)-1,2-phenylene diamine (18, 35). A 75% yield of a purple solid mp > 320 °C. ¹H NMR (DMSO-d₆/D₂O): 8.09 (d, 4H, J = 8), 8.06 (s, 2H), 7.83 (d, 2H, J = 8.4), 7.65 (d, 2H, J = 8.4), 7.48 (d, 4H, J = 8), 4.03 (br m, 2H), 3.07 (br, 4H), 1.29 (d, 12H, J = 6). ¹³NMR (DMSO-d₆/D₂O): 162.4, 153.0, 146.6, 138.3, 135.4, 129.8, 127.9, 124.8, 124.1, 123.5, 115.5, 115.0, 45.6, 36.2, 21.2. MS (FAB) 583 (M⁺+1) Anal. calc. for C₃₆H₃₈N₈•4HCl•0.5 H₂O: C, 58.61; H, 5.87; N, 15. 19. Found: C, 58.31; H, 5.79; N, 15.02.

### Example 17

### 4,4'-Bis[2-(5-N-cyclopentylamidino)benzimidazoyl]-2,5-diphenylfuran hydrochloride (Compound 54).

2,5-Bis(4-formylphenyl) furan was prepared by reduction of 2,5-bis-(4-cyanophenyl) furan (Bajic, M., (1996) *Heterocyclic Com.* 2: 135-140) (1.12 g 0.004 mol) using 1 M DIBAL in CH₂Cl₂ (1.83 g, 0.012 mol) to yield a pale yellow solid, 0.77 g (70%), mp 173-4 °C (CHCl₃: ether). ¹H NMR (DMSO-d₆/D₂O) 10.0 (s, 2H), 8.05 (d, 4H, J = 7.5), 7.97 (d, 4H, J = 7.5), 7.37 (s, 2H). ¹³C NMR (DMSO-d₆/D₂O) 192.0, 125.7, 135.0, 134.6, 130.1, 123.9, 111.6. MS m/e 276. Anal. calc. for C₁₈H₁₂O₃ :C, 91.49; H, 5.12. Found: C, 91.22; H, 5.38.

A procedure similar to that described in the above examples was employed for the condensation of 4-(N-cyclopentylamidino)-1,2-phenylene diamine with 2,5-bis(4-formylphenyl)furan to yield (77%) of a yellow solid mp 295-297 °C dec. ¹H NMR (DMSO-d₆/D₂O): 8.30 (d. 4H, J = 8.4), 8.05 (d, 4H, J = 8.4), 8.01 (s, 1H), 7.77 (d, 2H, J = 8.4), 7.56 (d, 2H, J = 8.4), 7.27 (s, 2H), 4.15 (br, 2H), 2.13-2.03 (m; 4H), 1.81-1.55 (m, 12H). ¹³C NMR (DMSO-d₆/D₂O): 163.7, 154.0, 153.1, 141.2, 138.6, 132.8, 127.9, 126.1, 124.6, 123.3, 123.1, 115.9,115.7,111.0,55.7,32.3,24.4. MS (FAB) m/z 673 (M⁺+1). Anal. calc. for C₄₂H₄₀N₈O•4HCl: C, 61.61; H, 5.42; N, 13.69. Found: C 62.28; H 5.74; N 13.62.

### Example 18

### 2,5-Bis[2-(5-amidino)benzimidazoyl]benzo[b]furan hydrochloride (Compound 55).

A procedure similar to that in the above-described examples was employed for the condensation of benzo[b]furan-2,5-dicarboxaldehyde and 4-amidino-1,2-phenylene diamine hydrochloride hydrate to give a 70% yield of a blue-gray solid mp 338-340°C dec. ¹H NMR (DMSO-d₆/D₂O) 8.6 (s, 1H), 8.27(d, 1H, J = 8), 8.19 (d, 2H, J = 9.6), 7.89 (d, 1H, J = 8.8), 7.87 (s, 1H), 7.83 (d, 1H, J = 8.4), 7.78 (d, 1H, J = 8.4). 7.73 (d, 1H, J = 8.8), 7.68 (d, 1H, J = 8). ¹³C NMR (DMSD-d₆/D₂O) 166.4, 165.9, 156.6, 153.5, 147.4, 145.4, 141.6, 139.9, 139.1, 136.9, 128.6, 126.2, 123.3, 122.9, 122.6, 122.2, 122.1,116.9, 115.8, 115.5, 115.0, 112.8, 108.6. MS(FAB) 435 (M⁺+1). Anal. calc. for C₂₄H₁₈N₈O•3HCl•H₂O: C, 51.30; H, 4.12; N, 19.94. Found: C, 51.72; H, 4.14; N, 15.64.

### Example 19

### 2,5-Bis[2-(5-N-cyclopentylamidino)benzimidazoyl]benzo[b]furan hydrochloride (Compound 56)

2-Acetyl-5-bromobenzo[b]furan was prepared as reported in the literature (Dann, O., (1972) *Liebigs Ann. Chem.,* 760:37-87) in an 86% yield; mp 110-111 °C (lit = 108-111 °C)¹H NMR (DMSO-d₆): 8.02 (d, 1H, J = 1.6), 7.79 (s, 1 H), 7.68(d, 1H, J = 9.2), 7.65 (dd, 1H, J = 9.2, 1.6), 2.58 (s, 3H). ¹³C NMR (DMSO-d₆): 181.6, 153.5, 152.9, 130.9, 128.8, 125.7, 115.9, 114.1, 112.9, 26.2. MS m/z 239(M⁺). The acetyl compound was converted into 5-bromobenzo[b]furan-2-carboxylic acid in a 77% yield as reported (15) mp 258-262 °C (lit = 258-262 °C). ¹H NMR (DMSO-d₆/D₂O): 7.95 (d, 1H, J = 1.6), 7.64 (d, 1H, J = 8.8), 7.58 (dd, 1H, J = 8.8, 1.6), 7.57 (s, 1H). ¹³C NMR (DMSO-d₆/D₂O): 159.5, 153.6, 147.3, 129.9, 128.9, 125.2, 115.8, 113.9, 112.5. MS m/z 241 (M⁺). 5-Bromobenzo[b]furan-2-carboxylic acid, used without further characterization, was converted, using standard procedures via the acid chloride, into 5-bromo-2-carboxamidobenzo[b]furan in a 81% yield; mp 208-210°C. ¹H NMR (DMSO-d₆): 8.2 (br s, 1H), 7.99 (s, 1H), 7.77 (br s, 1 H), 7.61 (d, 1H, J = 8.8), 7.57(dd, 1H, J = 8.8, 2) 7.51 (s, 1H). ¹³C NMR (DMSO-d₆/D₂O): 159.4, 153.0, 150.5, 129.4, 128.3, 125.2, 115.9, 113.9, 109.0. MS m/e 240 (M⁺). The amide, used without further characterization, was dehydrated employing POCI₃ to form 5-bromo-2-cyanobenzo[b]furan in a 88% yield; mp 147-148 °C. ¹H NMR (DMSO-d₆) 8.0 (s, 1H), 7.97 (s, 1H), 7.67 (br s ,2H). ¹³C NMR (DMSO-d₆) 153.7, 131.1 127.2, 127.1, 125.2, 118.8, 116.7, 113.7, 111.1. MS m/e 222(M⁺). 5-Bromo-2-cyanobenzo[b]furan, used without further characterization, was converted via a standard procedure (Newman, M.S., (1961) J. *Org. Chem.,* 26:2525) into 2,5-dicyanobenzo[b]furan in a 79% yield; mp 166-167 °C. ¹H NMR (DMSO-d₆): 8.39 (s, 1H), 8.14 (s, 1H), 7.97 (d, 1H, J = 8.8) 7.92 (d, 1H, J = 8.8). ¹³C NMR (DMSO-d₆): 156.3, 131.5 128.5, 128.2, 125.8, 119.3, 117.9, 113.4, 110.8, 107.7. MS m/e 168 (M⁺). Anal. calc. for C₁₀H₄N₂O: C, 71.42; H, 2.39; N, 16.16. Found: C, 71.78; H, 2.46; N,16.51. 2,5-Diformylbenzo[b]furan was prepared by reduction of the bis-nitrile using DIBAL which was added dropwise to a stirred solution of the bis-nitrile (1.68 g, 0.01 mol) in 150 ml dry methylene chloride under nitrogen. The mixture was stirred for 15 min, allowed to reflux for 40 min and the mixture was cooled. 100 ml of 1 M H₂SO₄ was added dropwise while maintaining the solution temperature below 25°C. The methylene chloride layer was separated, the aqueous layer was extracted with 100 ml of methylene chloride, and the combined organic phases were washed with 20% NaHCO₃, and dried over Na₂SO₄. The solvent was removed under reduced pressure and the residue was triturated with 1:1 ether. hexane. The resulting white solid was filtered, dried in a vacuum to yield 1.2 g (69%) mp 141-142 °C. ¹H NMR (DMSO-d₆): 10.1 (s, 1H), 9.92 (s, 1 H), 8.48 (d, 1H, J = 0.8), 8.10 (s, 1H), 8.08 (dd, 1H, J = 0.8, 8.88), 7.90 (d, 1H, J = 8.8). ¹³C NMR (DMSO-d₆): 191.8, 180.6, 158.1, 153.6, 132.9, 128.9, 127.5, 126.9, 118.8, 113.1. Anal. calc. for C₁₀H₆N₂•0.2 H₂O: C, 67.75; H, 3.67. Found: C, 67.83; H, 3.59. A protocol similar to that described above was employed for the condensation of 4-(N-cyclopentylamidino)-1,2-phenylene diamine with benzo[b]furan-2,5-dicarboxaldehyde to yield a gray solid in a 73% yield; mp 290-292 °C dec. ¹H NMR (DMSO-d₆/D₂O): 8.62 (s, 1H). 8.27(d, 1H. J = 8.8), 8.08 (s, 1H), 8.05 (s, 1H), 7.95 (d, 1H, J = 8.8), 7.90 (s, 1H), 7.84 (d, 1H, J = 8.8), 7.79 (d, 1H, J = 8.4), 7.64 (d, 1 H, J = 8.4). 7.57 (d, 1 H, J = 8.8), 4.14 (br 2H), 2.13-2.06
(m, 4H), 1.81-1.56 (m, 12H). ¹³C NMR (DMSO-d₆/D₂O): 163.4, 163.1, 156.9, 153.2, 147.6, 145.2, 141.2,138.8, 136.2, 128.9, 126.5, 124.5, 124.1 123.9, 123.5 122.5, 122.4, 116.9, 116.1, 115.8, 115.7, 115.1, 113.2, 108.8, 54.9, 54.8, 31.7, 23.9. MS(FAB) m/z 571 (M⁺+1). Anal. calc. for C₃₄H₃₄N₈O•4HCl: C, 56.99; H, 5.34; N, 15.64. Found: C, 56.89; H, 5.34; N, 15.53.

### Example 20

### 2,7-Bis[2-(5-N-isopropylamidino)benzimidazoyl]fluorene hydrochloride (Compound 57).

A mixture of 2,7-dibromofluorene 6.48 g (0.02 mol) and cuprous cyanide 5.37 g (0.06 mol) in 35 ml quinoline was heated under reflux for 2 h (followed by Thin Layer Chromatography, silica gel Kodak), benzene mobile phase). The mixture was cooled and extracted with 2X 150 ml chloroform. The organic layer was stirred with 200 ml 2M•HCl for 2 h, washed with water, dried over anhydrous Na₂SO₄, filtered, dried, concentrated, redissolved in minimum amount of chloroform, and chromatographed over neutral alumina. The column was first eluted with hexane: ether (2:1) to remove residual quinoline and finally with ether: CHCl₃(1:1) to CHCl₃ to afford fluffy pale solid 2.72 g (63%) mp 282-4 °C of 2,7-dicyanofluorene. ¹H NMR (DMSO-d₆/D₂O): 8.16 (d, 2H, J = 8.4), 8.04 (s, 2H), 7.83 (d, 2H, J = 8.4), 4.07 (s, 2H). ¹³C NMR (DMSO-d₆/D₂O): 144.2. 143.3, 130.8, 128.5, 121.7, 118.5, 110.1, 36.0. MS m/z 216 (M⁺). Anal. calc. for C₁₅H₈N₂: C, 83.21; H, 3.72; N, 12.95. Found: C 83.21; H 3.78; N 12.99. To a stirred solution of 2,7- dicyanofluorene (2.16 g, 0.01 mol) in 150 ml of dry CH₂Cl₂ was added DIBAL (1 M in cyclohexane, 4.26 g, 0.03 mol) under nitrogen at room temperature. The suspension was heated at 40 °C for 1 h, cooled, 100 ml 1M H₂SO₄ was added dropwise and stirred for 1h, the precipitated yellow solid was filtered, recrystallized from CHCl₃: ether to yield 2,7- diformylfluorene as pale crystalline solid, mp 218-220 °C, 1.6 g (72%). ¹H NMR (DMSO-d₆/D₂O) 10.08 (s, 2H), 8.16 (d, 2H, J = 8.0), 8.11 (s, 2H), 7.95 (d, 2H, J = 8.0), 4.10 (s, 2H). ¹³C NMR (DMSO-d₆/D₂O) 191.9, 145.0, 144.7, 135.6, 128.5, 125.4, 121.1, 36.0. MS m/e 222 (M⁺). Anal. calc. for C₁₅H₁₀O₂•0.1 H₂O: C, 80.41; H, 4.49. Found: C, 80.32; H, 4.63. A procedure similar to that in the above-described examples was used for the condensation of 2,7-diformylfluorene and 4-(N-isopropyfamidino)-1,2-phenylene diamine to yield (72%) of a green solid mp 310-313 °C dec.¹H NMR (DMSO-d₆/D₂O): 8.53 (s, 2H), 8.34 (d, 2H, J = 8.4), 8.22 (d, 2H, J = 8.4), 8.11 (s, 2H), 7.85 (d, 2H, J = 8.4), 7.65 (d, 2H, J = 8.4), 4.23 (s, 2H), 4.09 (quintet, 2H, J = 6.4), 1.33 (d. 12H, J = 6.4). ¹³C NMR (DMSO-d₆/D₂O) 162.2, 153.1, 144.9, 143.5, 138.7, 136.3, 126.9, 125.7, 124.3, 123.6, 121.7, 115.6, 114.7, 45.3, 36.7, 21.1. MS m/z (FAB) 567 (M⁺+1). Anal. calc. for C₃₅H₃₄N₂•4HCl: C, 58.99; H, 5.37; N, 15.73. Found: C, 59.14; H, 5.59; N, 15.43.

### Examples 21-25

Compounds described in these examples were synthesized in the laboratories of the inventors. Stock solutions of 10,000 µg were made in sterile distilled water or dimethyl sulfoxide (DMSO). The solutions were filter sterilized by passage through a 0.22 µm Millex Durapore membrane filter, and stored at -70°C until use. The synthesis and physical properties for the following compounds are set forth in the following references: compounds 1-15 (Patrick, D.A., et al., *Eur.J. Med.Chem.,* 32: pp. 781-793 (1997)); compounds 16 and 17 (Das, B.P., et al., *J*. *Med. Chem.* 20: pp. 531-536 (1977)); compound 18 (Boykin, D.W., et al., *Biorg. and Med. Chem. Letters* 6: pp. 3017-3020 (1996)); compounds 19 and 25 (Trent, J.O., et al., *J*. *Med. Chem.* 39: pp. 4554-4562 (1996)); compounds 20-24 (Boykin, D.W., et al., J. *Med. Chem.,* 41: pp. 124-129 (1998)); compounds 48-63 (Czarny, A.W., et al., *J. Heterocyclic Chemistry,* **33:** pp. 1393-1397 (1996)), and compounds 64-67 (Tidwell, R.R., et al., *J. Med. Chem.,* 21: pp. 613-623 (1978). A brief description of the synthesis and physical properties of the other compounds (33-38, and 40-41) is as follows.

Melting points were recorded using a Thomas Hoover (Uni-Melt) capillary melting point apparatus or a Fischer-Johns apparatus and are uncorrected. ¹H NMR and ¹³C NMR spectra were recorded employing a Varian GX400 spectrometer and chemical shifts (δ) are in ppm relative to TMS and coupling constants (J) are reported in Hertz. Mass spectra were recorded on a VG Instrument 70-SE spectrometer (Georgia Institute of Technology, Atlanta, GA). IR spectra were recorded using a Michelson 100 instrument (Bomem, Inc.). Elemental analysis were obtained from Atlanta Microlab Inc. (Norcross, GA) and are within a tolerance of 0.4 of the theoretical values. All chemicals and solvents were Aldrich Chemical Co. or Fisher Scientific.

In Examples 21-25, to a suspension of 2,5-bis[4-carboxyphenyl]furan diacid chloride (0.69g, 0.002mol) in 75 ml dry CH₂Cl₂ a substituted diamine (0.002 mol) was added, the mixture was stirred at room temperature for 6 h. The solvent was removed by distillation, the residue was treated with ice-water, the pH of the mixture was adjusted to 10 with 2M NaOH and the separated solid was filtered, washed with cold water and crystallized from ether: CHCl₃ or ether:methanol to yield pale yellow solids in yields of 75-85%. The free base was converted to its HCl salt by treating with saturated ethanolic-HCl in yields of 75-90%.

### Example 21

### 2,5-Bis-[4-(3-(N-morpholinopropyl)carbamoyl)phenyl]furan dihydrochloride (Compound 33)

An 83% yield of yellow crystalline solid mp 140-142°C dec was obtained using a procedure described above. The following data was obtained: ¹H NMR (DMSO-d₆/D₂O) 7.93 (d, 4H, J = 8.4), 7.86 (d, 4H, J = 8.4), 7.15 (s, 2H), 3.86 (br m, 8H), 3.38 (br m, 12H), 3.16 (t, 4H, J = 7.6), 2.0 (quintet, 4H, J = 7.6). ¹³C NMR (DMSO-d₆/D₂O) 166.5, 152.7, 133.1, 132.4, 128.0, 123.4, 110.0, 63.3, 54.3, 51.3, 36.6, 23.4. MS m/e 560 (M⁺). Anal. calcd. for C₃₂H₄₀N₄O₅!2HCl C, 60.65; H, 6.68; N, 8.84. Found: C, 60.59; H, 6.77; N, 8.87.

### Example 22

### 2,5-Bis[4-(2-N,N-dimethylaminoethylcarbamoyl)phenyl]furan dihydrochloride (Compound 34)

The procedure used for Example 21 was followed and an 80% yield of pale yellow crystalline solid mp 189-191°C dec was obtained. The following data was obtained: ¹H NMR (DMSO-d₆/D₂O) 7.94 (d, 4H, J = 8.4), 7.84 (d, 4H, J = 8.4), 7.12 (s, 2H), 3.66 (t, 4H, J = 6.0), 3.30 (t, 4H, J = 6.0), 2.84 (s, 12H). ¹³C NMR (DMSO-d₆/D₂O) 167.4, 152.9, 132.9, 132.7, 128.4, 123.7, 110.4, 56.6, 43.1, 34.9. MS m/e 448 (M⁺). Anal. calcd. for C₂₆H₃₂N₄O₃!2HCl C, 59.88 ; H, 6.57 ; N, 10.74,. Found: C, 59.78; H, 6.66; N, 10.64.

### Example 23

### 2,5-Bis[4-(3-N,N-dimethylaminopropylcarbamoyl)phenyl]furan dihydrochloride (Compound 35)

The procedure used in Example 21 was followed and a 75% yield of yellow hygroscopic solid mp 205-208°C dec was obtained. The following data were also obtained: ¹H NMR (DMSO-d₆) 10.91 (br, 2H), 8.80 (b s 2H), 8.03 (d, 4H, J = 8.4), 7.92 (d, 4H, J = 8.4), 7.23 (s, 2H), 3.40 (q, 4H, J = 6), 3.15 (quintet, 4H, J = 5.2), 2.76 (s, 6H), 2.75 (s, 6H), 2.02 (quintet, 4H, J = 6.8). ¹³C NMR (DMSO-d₆/D₂O) 165.6, 152.4, 132.8, 131.9, 127.7, 122.9, 109.6, 54.3, 41.7, 36.2, 23.8. MS m/e 476 (M⁺). Anal. calcd. for C₂₈H₃₆N₄O₃!2HCl!0.5 H₂O C, 60.21; H, 7.03; N, 10.03. Found: C, 60.51; H, 7.13; N, 9.95.

### Example 24

### 2,5-Bis[4-(3-N-methyl-3-N-phenylaminopropytcarbamoyl)phenyl]furan dihydrochloride (Compound 36)

The procedure used for Example 21 was employed and a 78% yield of yellow solid, 85-88°C dec was obtained. The following data was also obtained: ¹H NMR (DMSO-d₆/D₂O) 7.93 (d, 4H, J = 8.4), 7.89 (d, 4H, J = 8.4), 7.58 (d, 2H, J = 8.8), 7.55-7.48 (m,6H), 7.37 (S, 2H), 7.18 (s, 2H), 3.59 (m, 4H), 3.35 (t, 4H, J = 7.2), 3.16 (s, 6H), 1.83 (quintet, 4H, J = 7.2). ¹³C NMR (DMSO-d₆/D₂O) 166.4, 152.7, 133.1, 132.4, 130.1, 130.0, 128.0, 123.4, 119.7, 109.9, 55.3, 43.6, 36.5, 25.3. MS m/e. Anal. calcd for: C₃₈H₄₀N₄O₃!2HCl!0.75H₂O C; 66.37 ; H, 6.34 ; N, 8.15. Found: C, 66.43; H, 6.34; N, 8.21.

### Example 25

### 2,5-Bis[4-(3-N,N⁸,N¹¹-trimethylaminopropylcarbamoyl)phenyl]furan dihydrochloride (Compound 37)

The procedure used in Example 21 was employed and an 85% yield of yellow solid mp 245-247°C dec was obtained. The following data were also obtained: ¹H NMR (DMSO-d₆/D₂O) 7.82 (d,4H, J = 8), 7.56 (d, 4H, J = 8), 7.07 (s, 2H), 3.5-3.48 (m, 4H), 3.03 (brm, 4H), 2.94 (s, 6H), 2.76 (s, 12H), 1.98 (quintet, 4H, J = 7.6). ¹³C NMR (DMSO-d₆/D₂O) 171.2, 152.8, 135.4, 131.2, 127.9, 123.8, 109.7, 54.9, 42.7, 38.7, 22.4. MS m/e 504 (M⁺). Anal. Calcd. For: C₃₀H₄₀N₄O₃!2HCl!0.5H₂O. C, 61.42 ; H, 7.38 ; N, 9.45. Found: C, 61.11; H, 7.27; N, 9.61.

### Example 26

### 2,5-Bis[3-amidinophenyl]furan dihydrochloride (Compound 38)

A suspension of the imidate ester dihydrochloride(0.87 g, 0.002 mol), prepared from 2,5-bis[3-cyanophenyl]furan in 30 ml absolute ethanol was saturated with dry ammonia at 0-5°C and the mixture was stirred at room temperature for 2 h. The solvent was removed and the solid was treated with ice-water, filtered, basified with cold aqueous 2 M NaOH and the off-white precipitate which formed was filtered. The solid was washed with water and dried to yield 0.49 g (80.6%), mp 198-200 C. The free base (0.3 g, 0.001 mol) was converted into the HCI salt by treating with ethanolic HCI to yield 0.35 g (90%) mp 221-224°C dec. the following data was obtained: ¹H NMR (DMSO-d₆/D₂O) 8.18 (br s, 2H), 8.10 (d, 2H, J = 7.6), 7.68 (t, 2H, J = 7.6), 7.64 (d, 2H, J = 7.6), 7.14 (s, 2H) . ¹³C NMR (DMSO-d₆) 166.1, 152.5, 131.3, 130.6, 129.3, 129.0, 127.4, 123.3, 110.5. MS (FAB) m/e 305 (M⁺+1). Anal. Calcd. For. C₁₈H₁₆N₄O!2HCl!0.25 H₂O. C, 56.55; H, 4.88; N, 14.67. Found: C, 56.75; H, 4.84; N, 14.26.

### Example 27

### 2,5-Bis-(3-(N-isopropylamidino)amidinophenyl]furan dihydrochloride (Compound 40)

The imidate ester hydrochloride (0.435 g, 0.001 mol), prepared from 2,5-bis[3-cyanophenyl]furan and isopropyl amine (0.124 g, 0.0022 mol) in 10 ml ethanol were stirred for 12 h and yielded, after standard work-up, 0.13 g (80%) of beige free base having a mp 180-181 C (crystallized from hexane:ether 3:1). The free base on treatment with saturated ethanolic HCl yielded 0.29 g (75%) crystalline solid, mp 225-227 C dec. The following additional data were also obtained: ¹H NMR (DMSO-d₆) 8.18-8.12 (m, 4H), 7.71-7.62 (m, 4H), 7.27 (s, 2H), 4.09 (quintet, 2H, J = 6.4),1.30 (d, 12H, J = 6.4). ¹³C NMR (DMSO-d₆) 161.6, 152.1, 130.4, 129.9, 129.7, 127.9, 127.3, 123.3, 110.1, 45.2, 21.2.MS (FAB) m/e 339 (M⁺+1). Anal. calcd. for: C₂₄H₂₈N₄O!2HCl!0.25 H₂O (465.92) C, 61.86; H, 6.59; N, 12.02. Found: C, 61.82; H, 6.27; N, 11.76.

### Example 28

### 2,5-Bis[3[(N-(2-dimethylaminoethyl)amidino]phenylfuran dihydrochloride (Compound 41)

A mixture of imidate ester hydrochloride (0.435 g, 0.001 mol), prepared from 2,5-bis[3-cyanophenyl]furan and 2-dimethylaminoethylamine (0.185g, 0.0021 mol) in 10 ml ethanol was stirred at room temperature for 12 h. Subsequent to standard work up, 0.33g (74%) crystalline solid mp 75-77°C was obtained. The free base (0.30 g, 0.00072 mol) on treatment with saturated ethanolic HCI yielded 0.35 g (82%) of hydrochloride salt (very hygroscopic) which was dried at 75°C for 24 h; mp 260-263°C dec. The following additional data were also obtained: ¹H NMR (DMSO-d₆) 8.28(brs,2H), 8.12 (d, 2H, J = 8), 7.72 (d, 2H, J = 8), 7.65 (t, 2H, J = 8), 7.21 (s,2H), 3.9 (t, 4H, J = 4.2), 3.50 (t, 3H, J = 4.2), 2.89 (s, 12H). ¹³C NMR (DMSO-d₆) 164.1, 152.4, 130.9, 130.3, 129.6, 128.8, 127.6, 123.7, 110.5, 54.5, 43.2, 38.4. MS (FAB) m/e 447 (M⁺ +1). Anal. calcd. for: C₂₆H₃₄N₆O!4HCl (592.44) C, 52.70; H, 6.46; N, 14.19. Found: C, 52.43; H, 6.42; N, 13.99.

### Examples 1-20

### Antifungal Test Results

Antifungal susceptibility testing for compounds set forth in Examples 1-20 is discussed hereinbelow. The fungi that were employed included two reference strains, C. *neoformans* var. *neoformans* H99 and *C. albicans* A39 and the following clinical isolates: (1) two strains of fluconazole-resistant C. *neoformans* var. *neoformans* (135.95 and 114.96); (2) two strains of C. *neoformans* var. *gattii* (119.95 and 114.95); (3) two strains of fluconazole-resistant *C. albicans* (102.96 and 103.96); and (4) one strain each of *Torulopsis glabrata* (142.91), *Candida parapsilosis* (111.96), *Candida krusei* (132.91), *C. tropicalis* (110.96), *Candida lusitaniae* (111.92), *Fusarium solani* (152.89), *Aspergillus fumigatus* (168.95), and *Aspergillus flavus* (112.96). Antifungal susceptibility testing was performed using an RPMI-1640 medium (Sigma Chemical Co., St. Louis, MO) with glutamine, without sodium bicarbonate, and buffered at pH 7.0 with 0.165 M morpholine- propanesulfonic acid (MOPS).

*ln vitro* susceptibility testing in the form of Minimum Inhibitory Concentration (MIC) experiments were performed using the broth macrodilution method according to the recommendations of the National Committee for Clinical Laboratory Standards (see 1995 manual). The only difference compared to the standardized procedure was the choice of drug dilutions ranging from 100 to 0.09 µg/ml. Briefly, this procedure specifies an inoculum grown at 35°C and adjusted to a concentration of 0.5-2.5 x 10³ CFU/ml with an incubation of the test performed at 35°C and reading at 48 hours for all yeasts except for *C. neoformans* which is interpreted at 72 hr. The MIC was defined as the lowest drug concentration which showed a visual turbidity less than or equal to an 80 percent inhibition compared to that produced by the growth control tube.

Minimum Fungicidal Concentration (MFC) experiments were adapted from a method by McGinnis (McGinnis, M.R., (1980) *"Susceptibility testing and bioassay procedure*", p. 431, Academic Press, Inc., New York). Briefly, 10 µl aliquots from tubes with growth inhibition were plated onto Sabouraud agar plates. The lowest drug concentration that yielded three or fewer yeasts colonies was recorded as the MFC.

Molds were tested by the National Committee for Clinical Laboratory Standards method cited above using the below-referenced modifications. Isolates were grown on Sabouraud dextrose agar at 30°C and subcultured twice to insure viability. After adequate sporulation occurred (4-12 days), conidia were harvested by flooding colonies with a sterile solution of 0.85 percent NaCl and 0.05 percent Tween 80 in sterile distilled water. Inocula were prepared using a hemocytometer for counting, then diluted with RPMI 1640 medium to obtain a final inoculum size of approximately 0.5-2.5 x 10³ CFU/ml. The inoculum size was verified by plating an aliquot of the inoculum. Tests were incubated at 30°C for 48-72 h or until growth in the control tube was visible. In each experiment, the quality control included the testing of C. *albicans* A 39 and C. *neoformans* H99 against fluconazole and amphotericin B.

### Examples 21-28

### Antifungal Test Results

Antifungal susceptibility testing for compounds set forth in Examples 21-28 is discussed hereinbelow. The fungi that were employed included two quality control reference strains, *C. neoformans* var. *neoformans* H99 and C. *albicans* A39 and the following clinical isolates: (1) four strains of C. *neoformans* var. *neoformans* (167.95; 135.95; 114.96; and 133.95) of which three were fluconazole-resistant >64 µg/ml (135.95; 114.96; and 133.95); (2) two strains of C. *neoformans* var. *gattii* (119.95 and 114.95); (3) two strains of fluconazole-resistant >64 µg/ml C. *albicans* (102.96 and 103.96); and (4) one strain each of C. *glabrata* (142.91), *Candida parapsilosis* (111.96), *Candida krusei* (132.91), *C. tropicalis* (110.96), *Candida lusitaniae* (111.92), *Fusarium solani* (152.89), *Aspergillus fumigatus* (168.95), *Rhizopus arrhizus* (117.89), *Paecilomyces lilacinus* (137.90), and *Aspergillus flavus* (112.96). Antifungal susceptibility testing was performed using an RPMI-1640 medium (Sigma Chemical Co., St. Louis, MO) with glutamine, without sodium bicarbonate, and buffered at pH 7.0 with 0.165 M morpholinepropanesulfonic acid (MOPS).

*In vitro* susceptibility testing in the form of Minimum Inhibitory Concentration (MIC) experiments were performed using the broth macrodilution method according to the recommendations of the National Committee for Clinical Laboratory Standards (see 1995 manual). The only difference compared to the standardized procedure was the choice of drug dilutions ranging from 100 to 0.09 µg/ml. Briefly, this procedure specifies an inoculum grown at 35°C and adjusted to a concentration of 0.5-2.5 x 10³ CFU/ml with an incubation of the test performed at 35°C and reading at 48 hours for all yeasts except for C. *neoformans* which is interpreted at 72 hr. The MIC was defined as the lowest drug concentration which showed a visual turbidity less than or equal to an 80 percent inhibition compared to that produced by the growth control tube.

Minimum Fungicidal Concentration (MFC) experiments were adapted from a method by McGinnis (McGinnis, M.R., (1980) *"Susceptibility testing and bioassay procedure",* p. 431, Academic Press, Inc., New York). Briefly, 10 µl aliquots from tubes with growth inhibition were plated onto Sabouraud agar plates. The lowest drug concentration that yielded 3 or fewer yeasts colonies was recorded as the MFC. Several drugs were also rescreened with a subculture of 100 µl aliquots from tubes with no growth and there was no change in MFC.

Molds were tested by the National Committee for Clinical Laboratory Standards method cited above using the below-referenced modifications. Isolates were grown on Sabouraud dextrose agar at 30°C and subcultured twice to insure viability. After adequate sporulation occurred (4-12 days), conidia were harvested by flooding colonies with a sterile solution of 0.85 percent NaCl and 0.05 percent Tween 80 in sterile distilled water. Inoculum suspensions were prepared using a hemocytometer for counting, then diluted with RPMI 1640 medium to obtain a final inoculum size of approximately 0.5-2.5 x 10³ CFU/ml. The inoculum size was verified by plating an aliquot of the final concentration. Tests were incubated at 30°C for 48-72 h or until growth in the control tube was visible. The reproducibility of the method was quality controlled by testing fluconazole and amphotericin B against C. *albicans* A39 and C. neoformans H99 in each experiment. MICs for fluconazole against C. albicans A39 and C. neoformans H99 were 0.25 µg/ml and 2 µg/ml respectively. MICs for amphotericin B were 1 µg/ml against both isolates.

Tables 1-9 illustrate the antifungal activity according to the methods of the invention. As shown therein, the compounds used in the methods of the invention are effective in combating fungal infections, particularly in comparison to amphotericin, fluconazole, and pentamidine.

In the specification, and examples there have been disclosed typical preferred embodiments of the invention and, although specific terms are employed, they are used in a generic and descriptive sense only and not for the purposes of limitation, the scope of the invention being set forth in the following claims:

Aspects, embodiments, features and variations of the invention are further illustrated by the following clauses:-
1. A method of combating a fungal infection in a subject in need of such treatment, comprising administering to the subject an effective fungal infection-combating amount of a compound [(I)-(VI)] selected from the group consisting of: 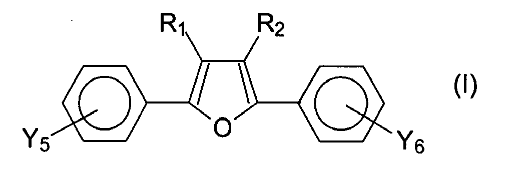 wherein R₁ and R₂ may be the same or different and selected from the group consisting of H, loweralkyl, aryl, alkylaryl, aminoalkyl, aminoaryl, halogen, oxyalkyl, oxyaryl, and oxyarylalkyl; and
   wherein Y₅ and Y₆ are present in the meta or para positions and may the same or different and are represented by the formula (a) or (b) selected from the group consisting of: 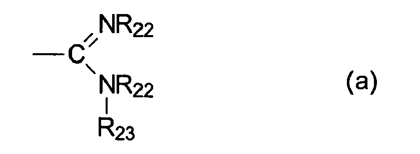 wherein:
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and 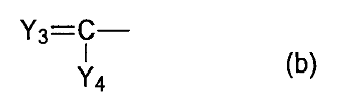 wherein Y₃ is selected from the group consisting of NR''' and O; wherein R''' is selected from the group consisting of H and loweralkyl;
      and wherein Y₄ is represented by the formula: 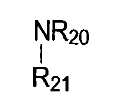 wherein R₂₀ is selected from the group consisting of H, loweralkyl, alkoxyalkyl, hydroxyalkyl, aminoalkyl, alkylaminoalkyl, cycloalkyl, aryl, or alkylaryl;
      wherein R₂₁ is selected from the group consisting of hydroxy, loweralkyl, alkoxyalkyl, hydroxyalkyl, aminoalkyl, alkylamino, alkylaminoalkyl, cycloalkyl, hydroxycycloalkyl, alkoxycycloalkyl, aryl, and alkylaryl; 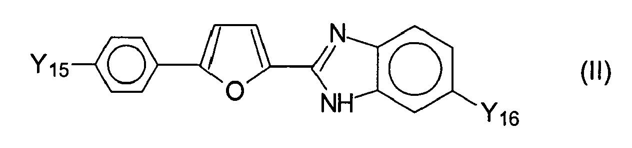 wherein Y₁₅ and Y₁₆ may be the same or different and represented by the formula: 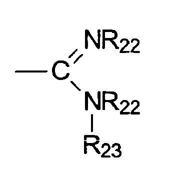 wherein:
   each R₂₂ is independently selected from the group consisting of H, loweralkyl, alkoxyalkyl, hydroxyalkyl, aminoalkyl, alkylaminoalkyl, alkylamino, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 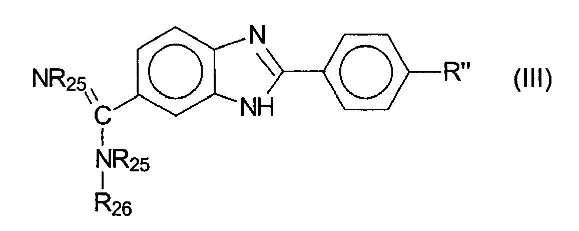 wherein each R₂₅ is independently selected from the group consisting of H, loweralkyl, alkoxyalkyl, hydroxyalkyl, aminoalkyl, alkylaminoalkyl, cycloalkyl, aryl, or alkylaryl or two R₂₅ groups together represent substituted or unsubstituted C₂-C₁₀ alkyl, hydroxyalkyl, or alkylene; and
      R₂₆ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
      R" is hydroxy, alkoxyalkyl, hydroxyalkyl, alkoxyaryl, aryl, or the substituent selected from the formula (i) and (ii) consisting of: 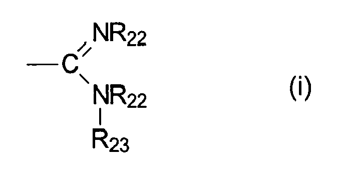 and 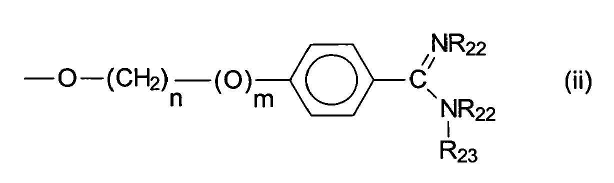 wherein:
   n and m may be independently selected and each range from 0 to 6;
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 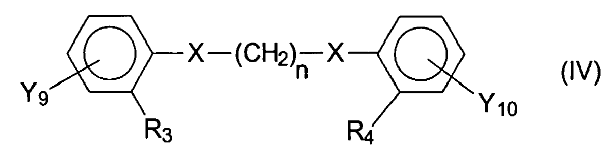 wherein:
   n is from 2 to 6;
   X is selected from the group consisting of O, NH, and S;
   Y₉ and Y₁₀ may be in the meta or para position, are independently selected and are each represented by the formula: 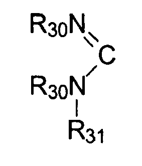
   wherein each R₃₀ is selected from the group consisting of H, hydroxy, loweralkyl, oxyalkyl, alkoxyalkyl, cycloalkyl, aryl, hydroxyalkyl, aminoalkyl, and alkylaminoalkyl; and wherein each of the two R₃₀ groups together may represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₃ and R₄ may be the same or different and are selected from the group consisting of H, amino nitro, loweralkyl, alkoxyalkyl, hydroxyalkyl, aminoalkyl, alkylaminoalkyl, cycloalkyl, aryl, or alkylaryl; 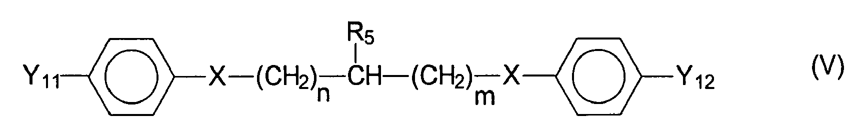
   wherein X may be O, NH, or S; n and m may be the same or different and range from 2 to 6;
   wherein Y₁₁ and Y₁₂ may be the same or different and represented by the formula: 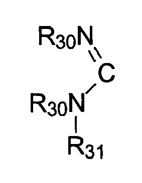
   wherein each R₃₀ is selected from the group consisting of H, loweralkyl, oxyalkyl, alkoxyalkyl, cycloalkyl, aryl, hydroxyalkyl, aminoalkyl, and alkylaminoalkyl; and wherein each of the two R₃₀ groups together may represent represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₅ is selected from the group consisting of H, hydroxy, and 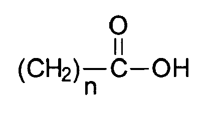
   wherein n ranges from 0 to 3; and 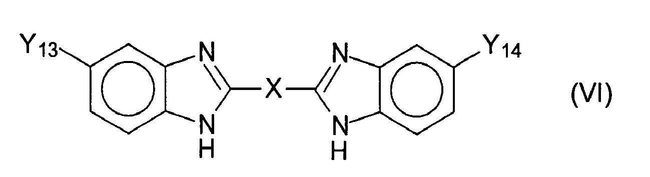
   wherein X is C₁ to C₁₂ linear or branched, saturated or unsaturated alkyl containing up to four double bonds, or is substituted or unsubstituted aryl;
   wherein Y₁₃ and Y₁₄ may be the same or different and are represented by the formula: 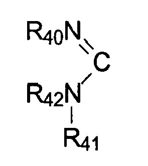
   wherein R₄₀ and R₄₂ are each independently selected from the group consisting of H, loweralkyl, cycloalkyl, substituted aryl, and unsubstituted aryl, or wherein R₄₀ and R₄₂ together may represent represent C₂-C₁₀ alkyl, hydroxyalkyl, alkylene, substituted aryl, or unsubstituted aryl;
   and wherein R₄₁ may be H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
2. A method according to clause 1, wherein said fungal infection is selected from the group consisting of *Candida albicans, Cryptococcus neoformans, Aspergillus fumigatus, Fusarium solani,* and combinations thereof.
3. A method according to clause 1, wherein said compound is represented by the formula: 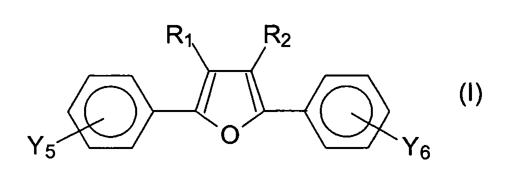 wherein R₁ and R₂ may be the same or different and selected from the group consisting of H, loweralkyl, aryl, alkylaryl, aminoalkyl, aminoaryl, halogen, oxyalkyl, oxyaryl, and oxyarylalkyl; and
   wherein Y₅ and Y₆ are present in the meta or para positions and may the same or different and are represented by the formula (a) or (b) selected from the group consisting of: 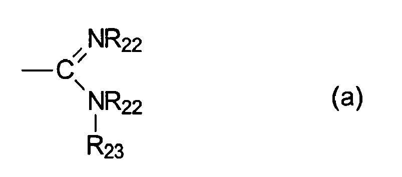 wherein:
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and 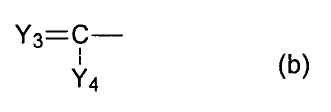 wherein Y₃ is selected from the group consisting of NR''' and 0; wherein R''' is selected from the group consisting of H and loweralkyl;
      and wherein Y₄ is represented by the formula: 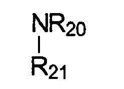 wherein R₂₀ is selected from the group consisting of H, loweralkyl, alkoxyalkyl, hydroxyalkyl, aminoalkyl, alkylaminoalkyl, cycloalkyl, aryl, or alkylaryl;
      wherein R₂₁ is selected from the group consisting of hydroxy, loweralkyl, alkoxyalkyl, hydroxyalkyl, aminoalkyl, alkylamino, alkylaminoalkyl, cycloalkyl, hydroxycycloalkyl, alkoxycycloalkyl, aryl, and alkylaryl.
4. A method according to clause 1, wherein said compound is represented by the formula: 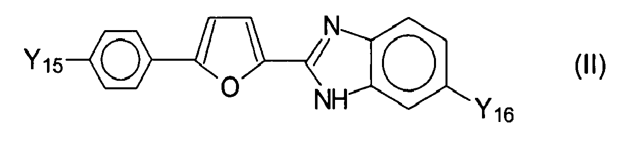 wherein Y₁₅ and Y₁₆ may be the same or different and represented by the formula: 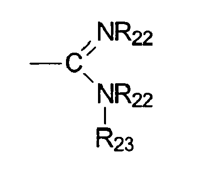 wherein:
   each R₂₂ is independently selected from the group consisting of H, loweralkyl, alkoxyalkyl, hydroxyalkyl, aminoalkyl, alkylaminoalkyl, alkylamino, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
5. A method according to clause 1, wherein said compound is represented by the formula: 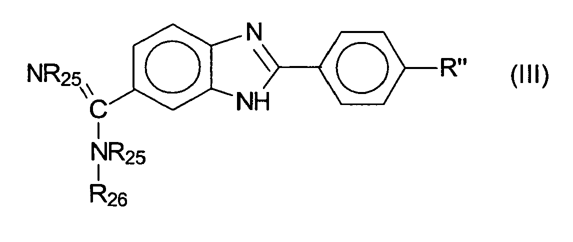 wherein each R₂₅ is independently selected from the group consisting of H, loweralkyl, alkoxyalkyl, hydroxyalkyl, aminoalkyl, alkylaminoalkyl, cycloalkyl, aryl, or alkylaryl or two R₂₅ groups together represent substituted or unsubstituted C₂-C₁₀ alkyl, hydroxyalkyl, or alkylene; and
   R₂₆ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   R" is hydroxy, alkoxyalkyl, hydroxyalkyl, alkoxyaryl, aryl, or the substituent selected from the formula (i) and (ii) consisting of: 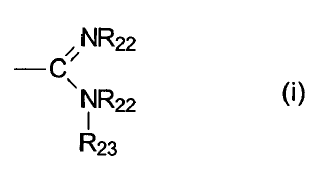 and 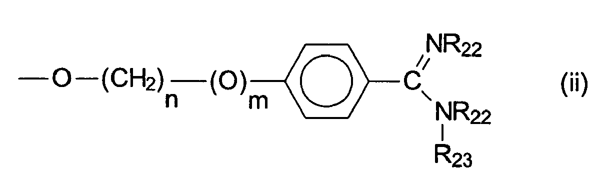 wherein:
   n and m may be independently selected and each range from 0 to 6;
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
6. A method according to clause 1, wherein said compound is represented by the formula: 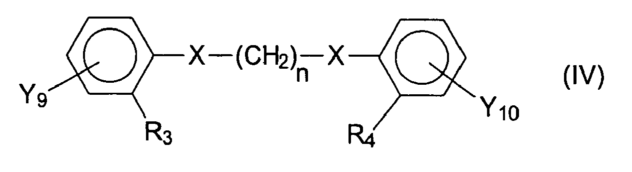 wherein:
   n is from 2 to 6;
   X is selected from the group consisting of O, NH, and S;
   Y₉ and Y₁₀ may be in the meta or para position, are independently selected and are each represented by the formula: 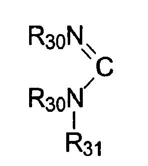
   wherein each R₃₀ is selected from the group consisting of H, hydroxy, loweralkyl, oxyalkyl, alkoxyalkyl, cycloalkyl, aryl, hydroxyalkyl, aminoalkyl, and alkylaminoalkyl; and wherein each of the two R₃₀ groups together may represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and
   wherein R₃ and R₄ may be the same or different and are selected from the group consisting of H, amino nitro, loweralkyl, alkoxyalkyl, hydroxyalkyl, aminoalkyl, alkylaminoalkyl, cycloalkyl, aryl, or alkylaryl.
7. A method according to clause 1, wherein said compound is represented by the formula: 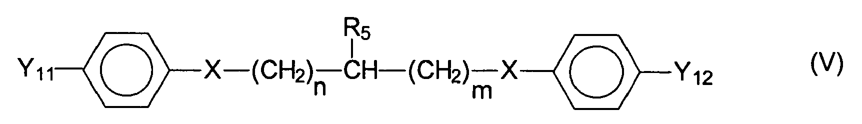 wherein X may be O, NH, or S; n and m may be the same or different and range from 2 to 6;
   wherein Y₁₁ and Y₁₂ may be the same or different and represented by the formula: 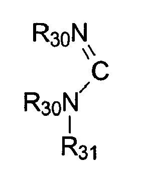 wherein each R₃₀ is selected from the group consisting of H, loweralkyl, oxyalkyl, alkoxyalkyl, cycloalkyl, aryl, hydroxyalkyl, aminoalkyl, and alkylaminoalkyl; and wherein each of the two R₃₀ groups together may represent represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and
   wherein R₅ is selected from the group consisting of H, hydroxy, and 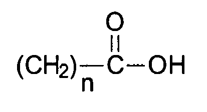 wherein n ranges from 0 to 3.
8. A method according to clause 1, wherein said compound is represented by the formula: 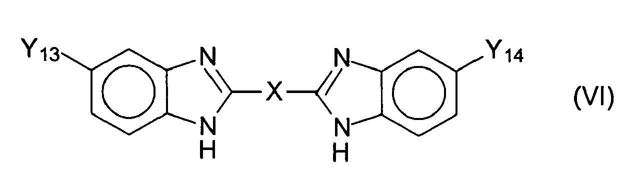 wherein X is C₁ to C₁₂ linear or branched, saturated or unsaturated alkyl containing up to four double bonds, or is substituted or unsubstituted aryl;
   wherein Y₁₃ and Y₁₄ may be the same or different and are represented by the formula: 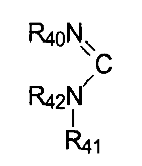 wherein R₄₀ and R₄₂ are each independently selected from the group consisting of H, loweralkyl, cycloalkyl, substituted aryl, and unsubstituted aryl, or wherein R₄₀ and R₄₂ together may represent represent C₂-C₁₀ alkyl, hydroxyalkyl, alkylene, substituted aryl, or unsubstituted aryl;
   and wherein R₄₁ may be H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
9. A method of combating a fungal infection selected from the group consisting of *Candida albicans* and *Cryptococcus neoformans* in a subject in need of such treatment, comprising administering to said subject an effective fungal infection-combating amount of a compound [(I)-(VI)] selected from the group consisting of: 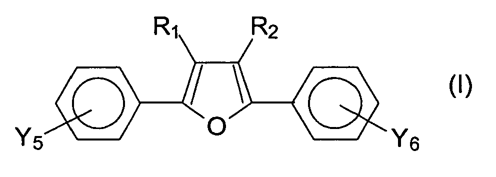 wherein R₁ and R₂ may be the same or different and selected from the group consisting of H, loweralkyl, aryl, alkylaryl, aminoalkyl, aminoaryl, halogen, oxyalkyl, oxyaryl, and oxyarylalkyl; and
   wherein Y₅ and Y₆ are present in the meta or para positions and may the same or different and are represented by the formula (a) or (b) selected from the group consisting of: 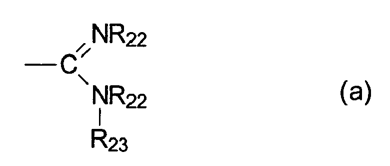 wherein:
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and 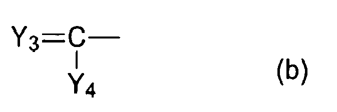
   wherein Y₃ is selected from the group consisting of NR''' and O; wherein R''' is selected from the group consisting of H and loweralkyl;
   and wherein Y₄ is represented by the formula: 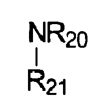 wherein R₂₀ is selected from the group consisting of H, loweralkyl, alkoxyalkyl, hydroxyalkyl, aminoalkyl, alkylaminoalkyl, cycloalkyl, aryl, or alkylaryl;
   wherein R₂₁ is selected from the group consisting of hydroxy, loweralkyl, alkoxyalkyl, hydroxyalkyl, aminoalkyl, alkylamino, alkylaminoalkyl, cycloalkyl, hydroxycycloalkyl, alkoxycycloalkyl, aryl, and alkylaryl; 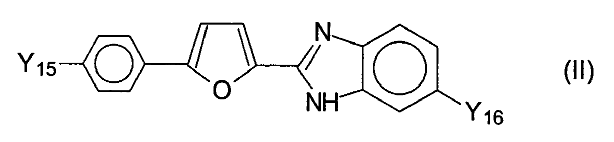 wherein Y₁₅ and Y₁₆ may be the same or different and represented by the formula: 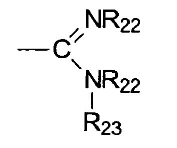 wherein:
   each R₂₂ is independently selected from the group consisting of H, loweralkyl, alkylamino, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 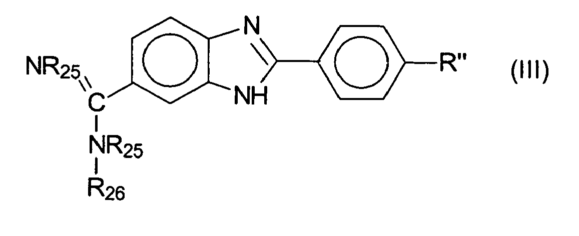
   wherein each R₂₅ is independently selected from the group consisting of H, loweralkyl, alkoxyalkyl, hydroxyalkyl, aminoalkyl, alkylaminoalkyl, cycloalkyl, aryl, or alkylaryl or two R₂₅ groups together represent substituted or unsubstituted C₂-C₁₀ alkyl, hydroxyalkyl, or alkylene; and
   R₂₆ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   R" is hydroxy, alkoxyalkyl, hydroxyalkyl, alkoxyaryl, aryl, or the substituent selected from the formula (i) and (ii) consisting of: 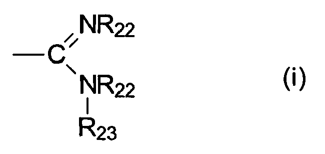 and 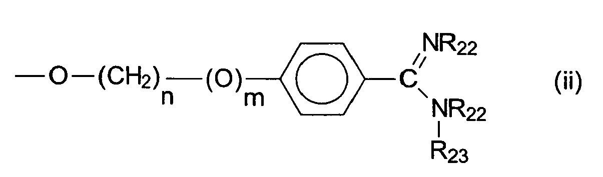 wherein:
   n and m may be independently selected and each range from 0 to 6;
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; 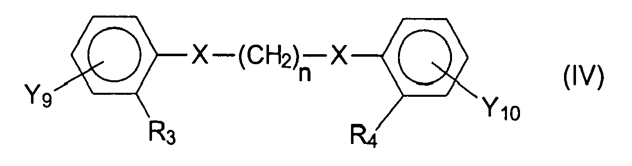
   wherein:
   n is from 2 to 6;
   X is selected from the group consisting of O, NH, and S;
   Y₉ and Y₁₀ may be in the meta or para position, are independently selected and are each represented by the formula: 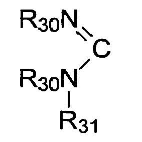
   wherein each R₃₀ is selected from the group consisting of H, hydroxy, loweralkyl, oxyalkyl, alkoxyalkyl, cycloalkyl, aryl, hydroxyalkyl, aminoalkyl, and alkylaminoalkyl; and wherein each of the two R₃₀ groups together may represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₃ and R₄ may be the same or different and are selected from the group consisting of H, amino nitro, loweralkyl, alkoxyalkyl, hydroxyalkyl, aminoalkyl, alkylaminoalkyl, cycloalkyl, aryl, or alkylaryl; 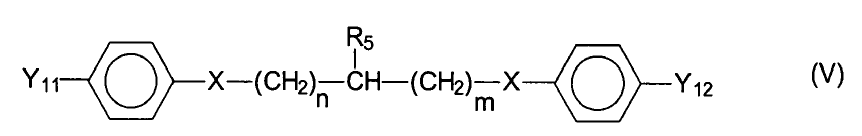
   wherein X may be O, NH, or S; n and m may be the same or different and range from 2 to 6;
   wherein Y₁₁ and Y₁₂ may be the same or different and represented by the formula: 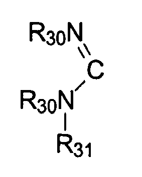
   wherein each R₃₀ is selected from the group consisting of H, loweralkyl, oxyalkyl, alkoxyalkyl, cycloalkyl, aryl, hydroxyalkyl, aminoalkyl, and alkylaminoalkyl; and wherein each of the two R₃₀ groups together may represent represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   wherein R₅ is selected from the group consisting of H, hydroxy, and 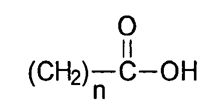
   wherein n ranges from 0 to 3; and 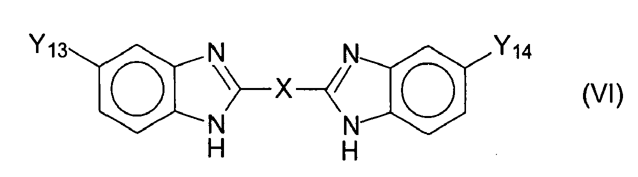
   wherein X is C₁ to C₁₂ linear or branched, saturated or unsaturated alkyl containing up to four double bonds, or is substituted or unsubstituted aryl;
   wherein Y₁₃ and Y₁₄ may be the same or different and are represented by the formula: 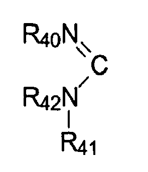
   wherein R₄₀ and R₄₂ are each independently selected from the group consisting of H, loweralkyl, cycloalkyl, substituted aryl, and unsubstituted aryl, or wherein R₄₀ and R₄₂ together may represent represent C₂-C₁₀ alkyl, hydroxyalkyl, alkylene, substituted aryl, or unsubstituted aryl;
   and wherein R₄₁ may be H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
10. A method according to clause 9, wherein said compound is represented by the formula: 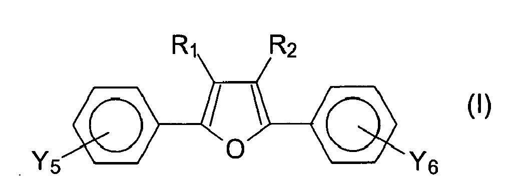 wherein R₁ and R₂ may be the same or different and selected from the group consisting of H, loweralkyl, aryl, alkylaryl, aminoalkyl, aminoaryl, halogen, oxyalkyl, oxyaryl, and oxyarylalkyl; and
   wherein Y₅ and Y₆ are present in the meta or para positions and may the same or different and are represented by the formula (a) or (b) selected from the group consisting of: 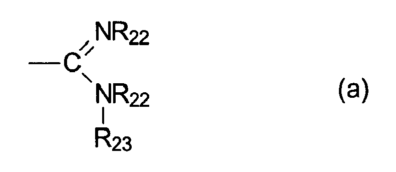 wherein:
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and 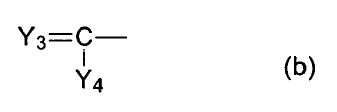 wherein Y₃ is selected from the group consisting of NR"' and O; wherein R"' is selected from the group consisting of H and loweralkyl;
      and wherein Y₄ is represented by the formula: 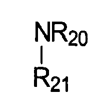 wherein R₂₀ is selected from the group consisting of H, loweralkyl, alkoxyalkyl, hydroxyalkyl, aminoalkyl, alkylaminoalkyl, cycloalkyl, aryl, or alkylaryl;
      wherein R₂₁ is selected from the group consisting of hydroxy, loweralkyl, alkoxyalkyl, hydroxyalkyl, aminoalkyl, alkylamino, alkylaminoalkyl, cycloalkyl, hydroxycycloalkyl, alkoxycycloalkyl, aryl, and alkylaryl.
11. A method according to clause 9, wherein said compound is represented by the formula: 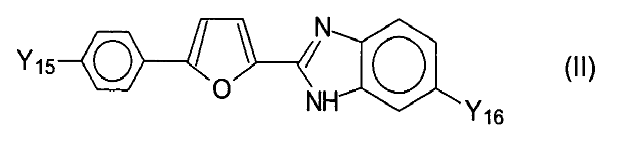 wherein Y₁₅ and Y₁₆ may be the same or different and represented by the formula: 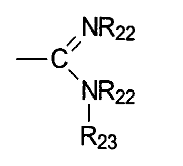 wherein:
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
12. A method according to clause 9, wherein said compound is represented by the formula: 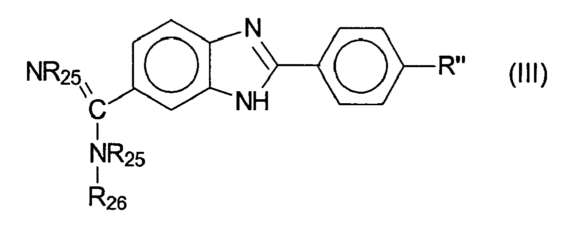 wherein each R₂₅ is independently selected from the group consisting of H, loweralkyl, alkoxyalkyl, hydroxyalkyl, aminoalkyl, alkylaminoalkyl, cycloalkyl, aryl, or alkylaryl or two R₂₅ groups together represent substituted or unsubstituted C₂-C₁₀ alkyl, hydroxyalkyl, or alkylene; and
   R₂₆ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   R" is hydroxy, alkoxyalkyl, hydroxyalkyl, alkoxyaryl, aryl, or the substituent selected from the formula (i) and (ii) consisting of: 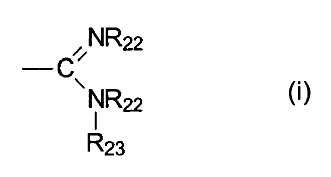 and 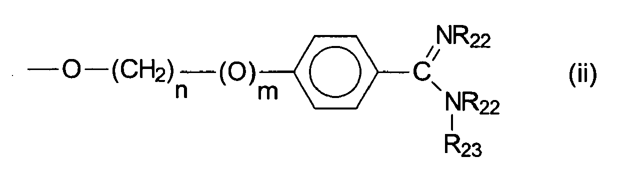 wherein:
   n and m may be independently selected and each range from 0 to 6;
   each R₂₂ is independently selected from the group consisting of H, loweralkyl, alkoxyalkyl, hydroxyalkyl, aminoalkyl, alkylaminoalkyl, cycloalkyl, aryl, or alkylaryl or two R₂₂ groups together represent C₂-C₁₀ alkyl, hydroxyalkyl, or alkylene; and R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
13. A method according to clause 9, wherein said compound is represented by the formula: 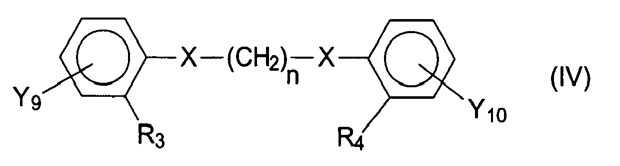 wherein:
   n is from 2 to 6;
   X is selected from the group consisting of O, NH, and S;
   Y₉ and Y₁₀ may be in the meta or para position, are independently selected and are each represented by the formula: 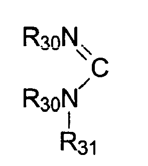
   wherein each R₃₀ is selected from the group consisting of H, hydroxy, loweralkyl, oxyalkyl, alkoxyalkyl, cycloalkyl, aryl, hydroxyalkyl, aminoalkyl, and alkylaminoalkyl; and wherein each of the two R₃₀ groups together may represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and
   wherein R₃ and R₄ may be the same or different and are selected from the group consisting of H, amino nitro, loweralkyl, alkoxyalkyl, hydroxyalkyl, aminoalkyl, alkylaminoalkyl, cycloalkyl, aryl, or alkylaryl.
14. A method according to clause 9, wherein said compound is represented by the formula: 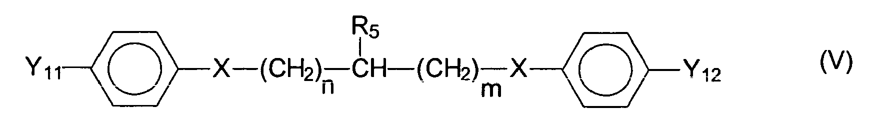 wherein X may be O, NH, or S; n and m may be the same or different and range from 2 to 6;
   wherein Y₁₁ and Y₁₂ may be the same or different and represented by the formula: 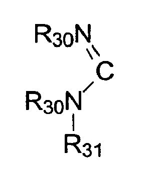 wherein each R₃₀ is selected from the group consisting of H, loweralkyl, oxyalkyl, alkoxyalkyl, cycloalkyl, aryl, hydroxyalkyl, aminoalkyl, and alkylaminoalkyl; and wherein each of the two R₃₀ groups together may represent represent C₂-C₁₀ alkyl, hydroxyalkyl, or alkylene;
   wherein R₃₁ is selected from the group consisting of H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl; and
   wherein R₅ is selected from the group consisting of H, hydroxy, and 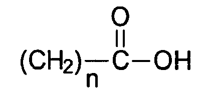 wherein n ranges from 0 to 3.
15. A method according to clause 9, wherein said compound is represented by the formula: 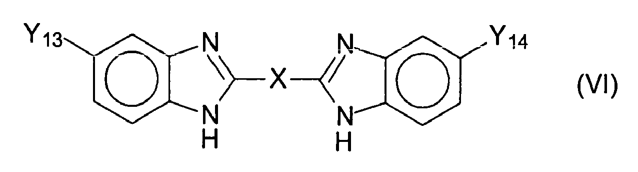 wherein X is C₁ to C₁₂ linear or branched, saturated or unsaturated alkyl containing up to four double bonds, or is substituted or unsubstituted aryl;
   wherein Y₁₃ and Y₁₄ may be the same or different and are represented by the formula: 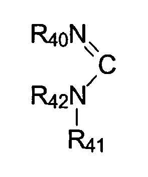 wherein R₄₀ and R₄₂ are each independently selected from the group consisting of H, loweralkyl, cycloalkyl, substituted aryl, and unsubstituted aryl, or wherein R₄₀ and R₄₂ together may represent represent C₂-C₁₀ alkyl, hydroxyalkyl, alkylene, substituted aryl, or unsubstituted aryl; and wherein R₄₁ may be H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl.
16. A compound for administering to a subject in need of fungal treatment represented by the formula: 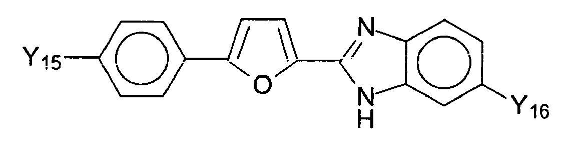 wherein Y₁₅ and Y₁₆ may be the same or different and represented by the formula: 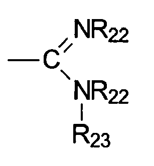 wherein:
   each R₂₂ is independently selected from the group consisting of H, loweralkyl, alkoxyalkyl, hydroxyalkyl, aminoalkyl, alkylamino, alkylaminoalkyl, cycloalkyl, aryl, or alkylaryl or two R₂₂ groups together represent C₂-C₁₀ alkyl, hydroxyalkyl, or alkylene;
   R₂₃ is H, hydroxy, loweralkyl, alkoxyalkyl, aminoalkyl, alkylamino, alkylaminoalkyl, cycloalkyl, hydroxycycloalkyl, alkoxycycloalkyl, aryl, or alkylaryl;
   and pharmaceutically acceptable salts thereof.

## Claims

1. Use, for the manufacture of a medicament for the treatment of fungai infections, of a compound [(i), (iii), or (Vi)], or a pharmaceutically acceptable salt thereof, selected from the group consisting of: 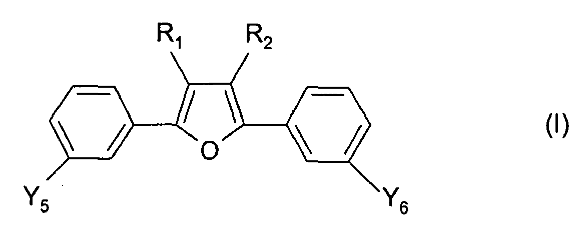 wherein R₁ and R₂ can be the same or different and are selected from the group consisting of H, C₁ to C₄ linear or branched alkyl, aryl, alkylaryl, aminoalkyl, aminoaryl, halogen, oxyalkyl, oxyaryl, and oxyarylalkyl; and wherein Y₅ and Y₆ can be the same or different and are represented by formula (a): 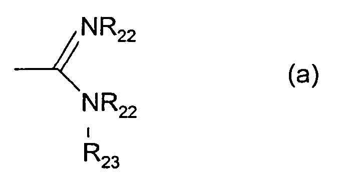 wherein:
each R₂₂ is H or two R₂₂ groups together represent C₂-C₁₀ alkyl, hydroxyalkyl, or alkylene; and
R₂₃ is H, hydroxy, C₁ to C₄ linear or branched alkyl, alkyoxyalkyl, aminoalkyl, alkylamino, alkylaminoalkyl, cycloalkyl, hydroxycycloalkyl, alkoxycycloalkyl, aryl, or alkyaryl; 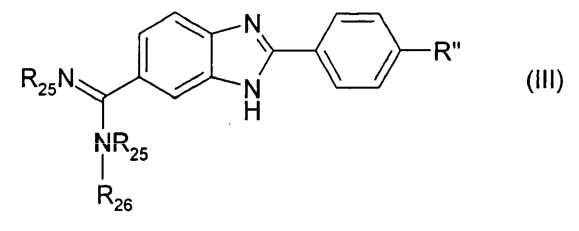
wherein each R₂₅ is independently selected from the group consisting of H, C₁ to C₄ linear or branched alkyl, alkoxyalkyl, hydroxyalkyl, aminoalkyl, alkylaminoalkyl, cycloalkyl, aryl, or alkylaryl, or two R₂₅ groups together represent substituted or unsubstituted C₂-C₁₀ alkyl, hydroxyalkyl, or alkylene; and R₂₆ is H, hydroxy, C₁ to C₄ linear or branched alkyl, alkoxyalkyl, aminoalkyl, alkylamino, alkylaminoalkyl, cycloalkyl, hydroxycycloalkyl, alkoxycycloalkyl, aryl, or alkylaryl;
R" is hydroxy, C₁ to C₄ linear or branched alkoxy, alkoxyalkyl, hydroxyalkyl, alkoxyaryl, aryl, or a substituent of formula (i): 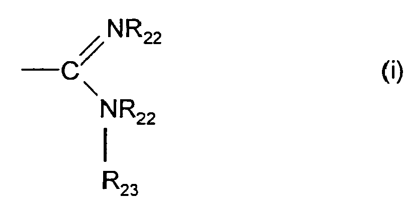
wherein:
each R₂₂ is independently selected from the group consisting of H, C₁ to C₄ linear or branched alkyl, alkoxyalkyl, hydroxyalkyl, aminoalkyl, alkylaminoalkyl, cycloalkyl, aryl, or alkylaryl, or two R₂₂ groups together represent C₂-C₁₀ alkyl, hydroxyalkyl, or alkylene; and R₂₃ is H, hydroxy, C₁ to C₄ linear or branched alkyl, alkoxyalkyl, aminoalkyl, alkylamino, alkylaminoalkyl, cycloalkyl, hydroxycycloalkyl, alkoxycycloalkyl, aryl, or alkylaryl; and 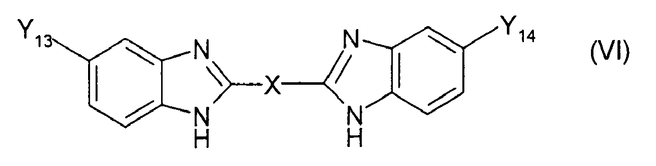
wherein X is C₁ to C₁₂ linear or branched, saturated or unsaturated alkyl containing up to four double bonds, or is substituted or unsubstituted aryl; wherein Y₁₃ and Y₁₄ can be the same or different and are represented by the formula: 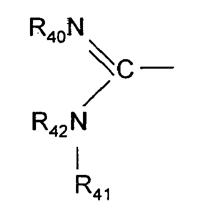
wherein R₄₀ and R₄₂ are each independently selected from the group consisting of H, C₁ to C₄ linear or branched alkyl, cycloalkyl, substituted aryl, and unsubstituted aryl, or wherein R₄₀ and R₄₂ together represent C₂-C₁₀ alkyl, hydroxyalkyl, alkylene, substituted aryl, or unsubstituted aryl; and wherein R₄₁ can be H, hydroxy, C₁ to C₄ linear or branched alkyl, alkoxyalkyl, aminoalkyl, alkylamino, alkylaminoalkyl, cycloalkyl, hydroxycycloalkyl, alkoxycycloalkyl, aryl or alkylaryl.

2. Use as claimed in Claim 1, wherein said fungal infection is selected from the group consisting of *Candida albicans, Cryptococcus neoformans, Aspergillus fumigatus, Fusarium solani,* and combinations thereof.

3. Use as claimed in Claim 1 or 2 wherein said fungal infection is selected from *Candida albicans* and *Cryptococcus neoformans.*

4. Use as claimed in Claim 1, 2 or 3 wherein said compound is 2,5-bis[3-amidinophenyl]furan or a pharmaceutically acceptable salt thereof.

5. Use as claimed in Claim 1, 2 or 3 wherein said compound is represented by the formula (III) or a pharmaceutically acceptable salt thereof.

6. Use as claimed in Claim 1, 2 or 3 wherein said compound is represented by the formula (VI) or a pharmaceutically acceptable salt thereof.

7. Use as claimed in Claim 6, wherein X is C₁ to C₁₂ linear or branched, saturated or unsaturated alkyl containing up to four double bonds or substituted or unsubstituted aryl, wherein the substituted or unsubstituted aryl is selected from the group consisting of phenyl, alkyl-substituted phenyl, phenylalkylphenyl, benzofuran, and fluorene.

8. Use as claimed in Claim 6 or 7, wherein said compound is selected from the group consisting of:
4,4'-Bis[2-(5-*N*-isopropylamidino)benzimidazoyl]-1,2-diphenylethane hydrochloride;
2,5-Bis[2-(5-amidino)benzimidazoyl]benzo[b]furan hydrochloride;
2,5-Bis[2-(5-N-cyclopentylamidino)benzimidazoyl]benzo[b]furan hydrochloride; and
2,7-Bis[2-(5-*N*-isopropylamidino)benzimidazoyl]fluorene hydrochloride.
